# EUROPEAN PATENT SPECIFICATION

(11) **EP 2 863 850 B1**
(45) Date of publication and mention of the grant of the patent: **01.11.2017**
(21) Application number: 13745158.9
(22) Date of filing: 24.06.2013
(51) Int. Cl.: A61F 7/02, A61F 7/00

(54) **THERMAL AND/OR PRESSURE REGULATION CONTROL SYSTEM**
WÄRME- UND/ODER DRUCKREGELSTEUERSYSTEM
SYSTÈME DE COMMANDE DE RÉGULATION THERMIQUE ET/OU DE PRESSION

(30) Priority: 22.06.2012 GB 201211149; 23.05.2013 GB 201309310
(43) Date of publication of application: 29.04.2015
(73) Proprietor: Physiolab Technologies Limited, Milton Keynes, Buckinghamshire MK11 3JB (GB)
(72) Inventor: ROSE, Nicholas, Milton Keynes Buckinghamshire MK11 3JB (GB); HASKINS, Mark, Milton Keynes Buckinghamshire MK11 3JB (GB)
(74) Representative: Williams Powell
(86) International application number: PCT/GB2013/051663
(87) International publication number: WO 2013/190336

(56) References cited:
- WO-A1-01/54635
- WO-A1-96/26693
- WO-A1-03/030790
- WO-A1-2012/020267
- US-A- 4 335 726
- US-A- 5 097 829
- US-A1- 2004 210 283
- US-B1- 6 508 831

## Description

### Technical Field

The present invention relates to a control system for a thermoregulation assembly used to apply thermal and/or compression treatment to a patient in order to alleviate or cure physical injuries and disorders.

### Background Art

There are numerous instances where it is desired to effect a thermal treatment on a patient alone or in combination with compression. For example, this may be to treat a physical injury, such as of the muscles, ligaments, tendons and the like. It may also be useful in treating skin injuries, as well as illnesses such as infections and so on as well as to accelerate natural recovery.

Thermal treatments of this type have been known for many years, in their simplest form being ice packs. Heat generating packs have also been developed, typically in the form of a pouch of chemical material which can be made to react exothermically and thereby to release heat. These devices are intended to cool or heat, as appropriate, a part of a person's body in order to alleviate inflammation, pain suffered by the patient and so on. It has been found that if properly applied, such treatments can significantly minimise patient discomfort caused by the injury or illness as well as speed up the recovery process. However, such ice packs and heat generating packs provide a relatively crude form of temperature regulation, unable to provide optimum treatment of an injury without constant and close monitoring by a medical practitioner.

More recently, attempts have been made to develop thermoregulation devices which have some form of built-in control, for example in which a desired treatment temperature can be set in a control unit and then used to feed energy to a cuff or pad. This may be by means of heated or cooled fluid or by electrical heating or cooling. An early example known to the applicant is DE-3,343,664. Other examples include EP-0,812,168, US-6,818,012 and EP-2,393,459.

While such control systems are known and have attempted to provide accurately controllable temperature regulation at the patient's skin, there are numerous variables which result in such systems being inaccurate. Moreover, in such systems, particularly fluid based systems, it has been found difficult to produce the desired temperature at the actual site of the patient to be treated. This is caused by a number of factors, of which the primary include difficulties in controlling the flow of fluid in an applicator cuff or garment, difficulties in ensuring proper energy transfer to the patient through the cuff, speed of energy transfer to the patient with consequential speed of adjustment of treatment parameters, and so on. If these difficulties could be overcome, it is believed that fluid based systems could provide a very effective form of thermoregulation.

The prior art does not generally resolve the problems indicated above.

### Summary of the Present Invention

The present invention seeks to provide an effective control system for a thermoregulation assembly, an effective pressure regulation assembly for the treatment of physical injuries and disorders and for the speeding up of natural recovery.

According to an aspect of the invention, there is provided a control system as in claim 1.

According to an aspect of the invention, there is provided a program product as in claim 9.

Described herein is a control system for a thermoregulation assembly, including:
a fluid manifold assembly including a manifold input for receiving thermoregulation fluid from at least one source of thermoregulation fluid at a thermoregulation temperature, and a manifold output for supplying thermoregulation fluid to an interface pack; the manifold assembly being operable to selectively provide thermoregulation fluid into a said interface pack via the manifold output in accordance with feed parameters;
a control unit operable to obtain a desired temperature profile, the desired temperature profile providing a profile of desired interface pack temperature; and
a second input, the second input being for receiving thermoregulation fluid from a said interface pack and an input temperature sensor coupled to the control unit and disposed to measure a return temperature of thermoregulation fluid received at the second input from a said interface pack;
wherein the control unit is operable to repeatedly determine, based at least on the return temperature sensed by the input temperature sensor, an interface pack temperature;
wherein the control unit is operable to modify one or more feed parameters in response to the interface pack temperature to bring the interface pack temperature into correspondence with the desired temperature profile;
wherein the control unit is operable to operate the fluid manifold assembly in accordance with said one or more feed parameters;

Described herein is a method of operating a thermoregulation assembly, the assembly including:
a control unit;
an interface pack provided with at least one fluid path therein for the passage of thermoregulation fluid therethrough and a fluid inlet and a fluid outlet;
a fluid manifold assembly including a manifold output attachable in fluid communication with the fluid inlet of the interface pack, the manifold assembly being operable to selectively provide thermoregulation fluid into the fluid inlet of the interface pack in accordance with feed parameters;
at least one source of thermoregulation fluid at a thermoregulation temperature coupled to a manifold input for supplying thermoregulation fluid to the manifold assembly;
a second input for receiving thermoregulation fluid from the interface pack and an input temperature sensor coupled to the control unit and disposed to measure a return temperature of thermoregulation fluid received at the second input from the interface pack;
the method including the steps of:
   obtaining a desired temperature profile, the desired temperature profile providing a profile of desired interface pack temperature;
   operating the fluid manifold assembly in accordance with feed parameters;
   repeatedly determining, based at least on the return temperature sensed by the input temperature sensor, an interface pack temperature;
   modifying one or more feed parameters in response to the interface pack temperature to bring the interface pack temperature into correspondence with the desired temperature profile.

Described herein is a method of operating a thermoregulation assembly, the assembly including:
a control unit;
an interface pack provided with at least one fluid path therein for the passage of thermoregulation fluid therethrough and at least one fluid inlet and at least one fluid outlet;
a fluid manifold assembly including a coupling attachable in fluid communication with the interface pack, the manifold assembly including at least one controllable valve for selectively feeding thermoregulation fluid into the interface pack;
at least one source of thermoregulation fluid at a thermoregulation temperature, the source including at least one fluid temperature variation unit;
a fluid pump coupled between the at least one source of thermoregulation fluid and the to the manifold assembly, and coupled to the control unit;
a thermoregulation treatment input unit coupled to the control unit; and
at least inlet and outlet temperature sensors disposed to measure temperature at the fluid inlet and fluid outlets respectively and coupled to the control unit;
the method including the steps of:
   obtaining a measure a patient's body temperature on the basis of the temperature sensed by the outlet temperature sensor;
   determining a deviation between the sensed patient temperature and a desired or set temperature;
   activating the control unit to operate the pump so as to pump thermoregulation fluid into the interface pack through the manifold assembly;
   obtaining a measure the temperature of the thermoregulation fluid at the fluid outlet;
   determining a measure of the patient's heat absorption rate by determining a difference in the inlet and outlet temperatures over time;
   adjusting the temperature of the thermoregulation fluid or length of time of operation of the pump on the basis of the determined patient's heat absorption rate.

In some embodiments, the thermoregulation fluid is water.

Embodiments of the invention relate to bio-mechanical tuning. In some cases in this description and accompanying drawings, bio-mechanical tuning is referred to as 'bio tune'.

Other preferred features of the teachings herein will become apparent from the specific description which follows.

### Brief Description of the drawings

Embodiments of the present invention are described below, by way of example only, with reference to the accompanying drawings, in which:
Figure 1 is a schematic diagram of a preferred embodiment of manifold assembly provided in a thermoregulation system;
Figure 2 shows the system of Figure 1 with the manifold configured to supply 50% coolant and 50% heating fluid, with return fluid being fed into the coolant and heating supplies;
Figure 3 shows the system of Figure 1 with the manifold configured to supply 25% coolant, 0% heating fluid, with return fluid being recirculated into the supply chamber;
Figure 4 shows the system of Figure 1 with the manifold configured to recirculate all fluid within the manifold without passing the fluid into the garment or cuff;
Figure 5 shows the system of Figure 1 with the manifold configured to recirculate all fluid into the garment or cuff;
Figure 6 is a schematic diagram of a flowchart explaining operation of a manifold assembly;
Figure 7 is a schematic diagram of an example of an interface pack;
Figure 8 is a schematic diagram of part of another example of an interface pack;
Figure 9 is a schematic diagram of a manifold assembly according to an embodiment of the invention;
Figure 10 is a schematic diagram of a mixing chamber for use in embodiments of the invention;
Figure 11 is an explanatory diagram according to embodiments of the invention;
Figures 12, 14 and 15 are flow diagrams according to embodiments of the invention;
Figure 13 is a schematic diagram of an interface pack used in embodiments of the invention;
Figure 16 is an illustrative graph for an embodiment of the invention;
Figures 17 and 18 are explanatory diagrams according to embodiments of the invention;
Figures 18a to 20 include graphs illustrating features of the operation according to embodiments of the invention;
Figure 21 are exemplary look-up graphs for use in embodiments of the invention;

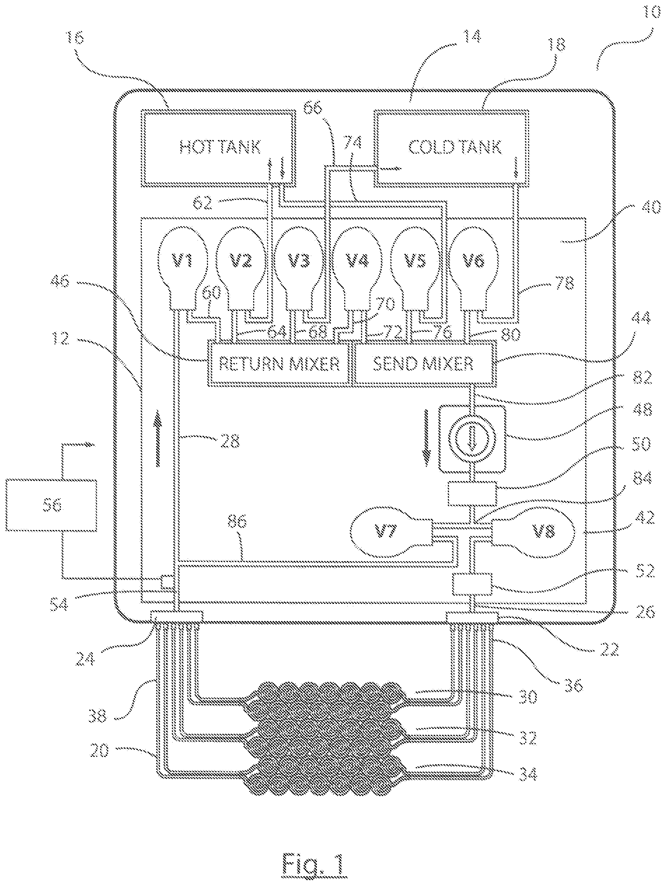

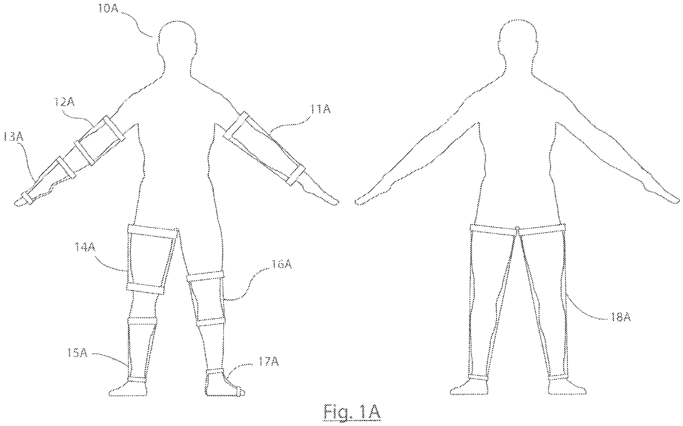

Figure 1A is a schematic diagram of a patient wearing a number of interface packs usable in embodiments of the invention;

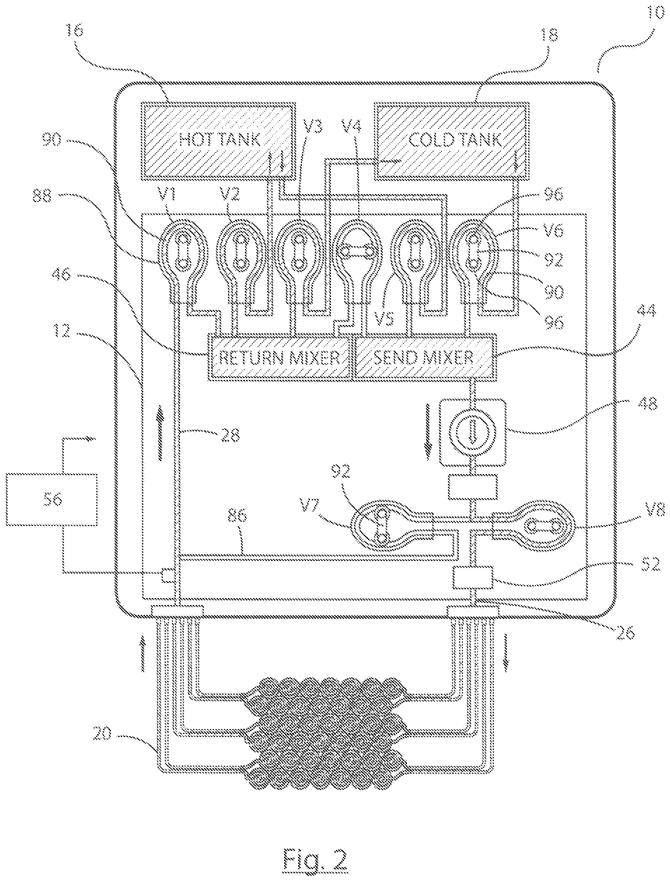

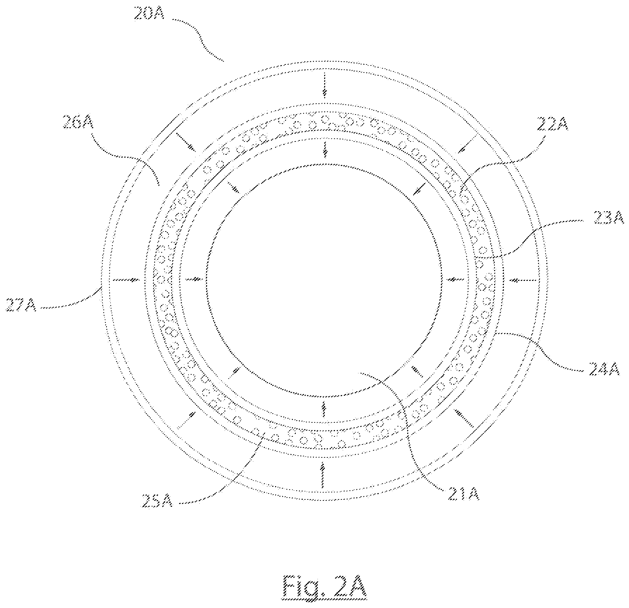

Figure 2A is a cross-sectional schematic diagram of an interface pack usable in embodiments of the invention worn on a body part;

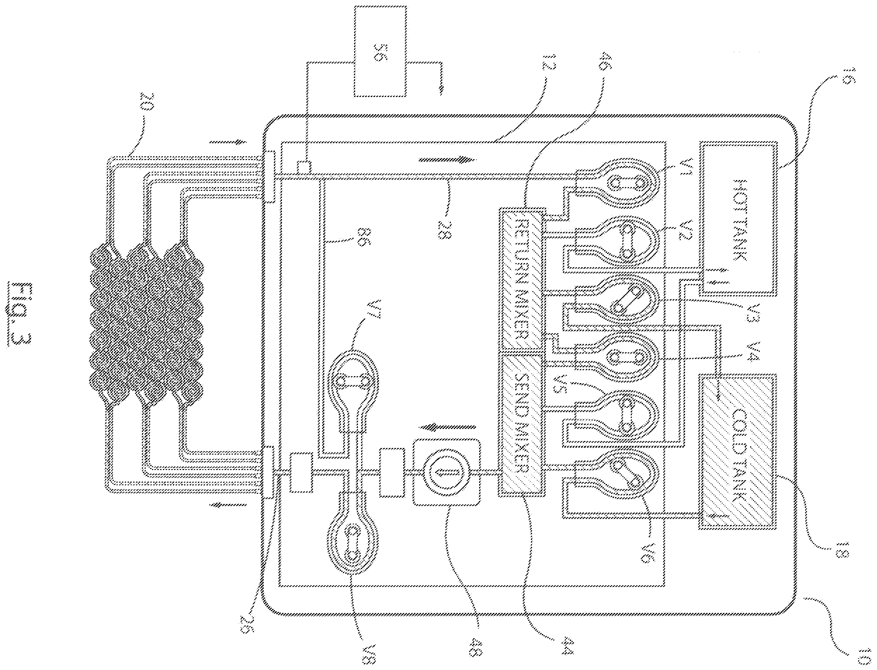

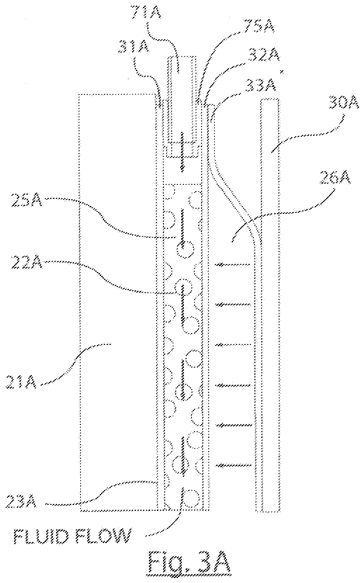

Figure 3A is a schematic longitudinal cross-section of a part of an interface pack usable in embodiments of the invention;

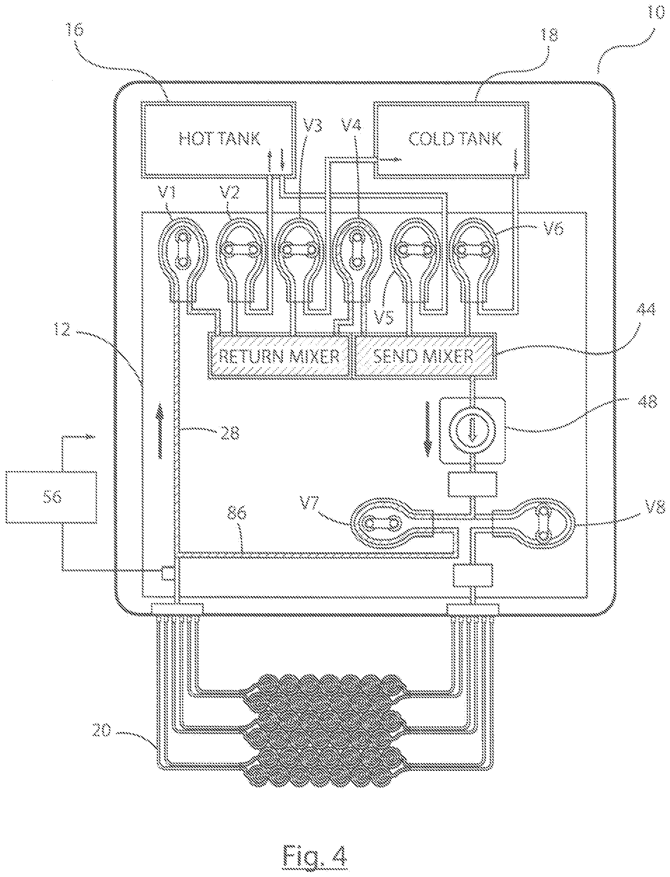

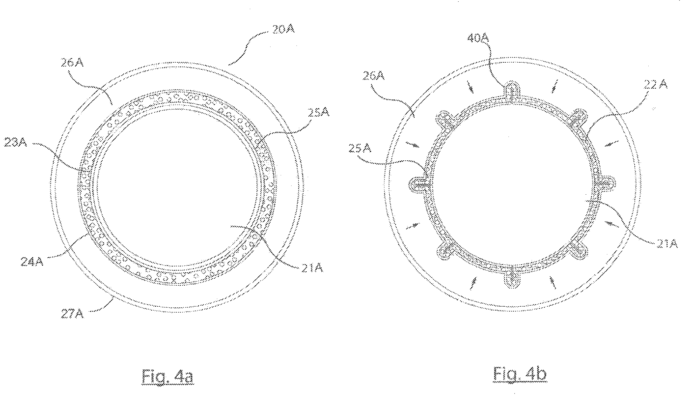

Figures 4a and 4b are schematic cross-sectional diagrams of an interface pack usable in embodiments of the invention under no compression and under compression respectively;

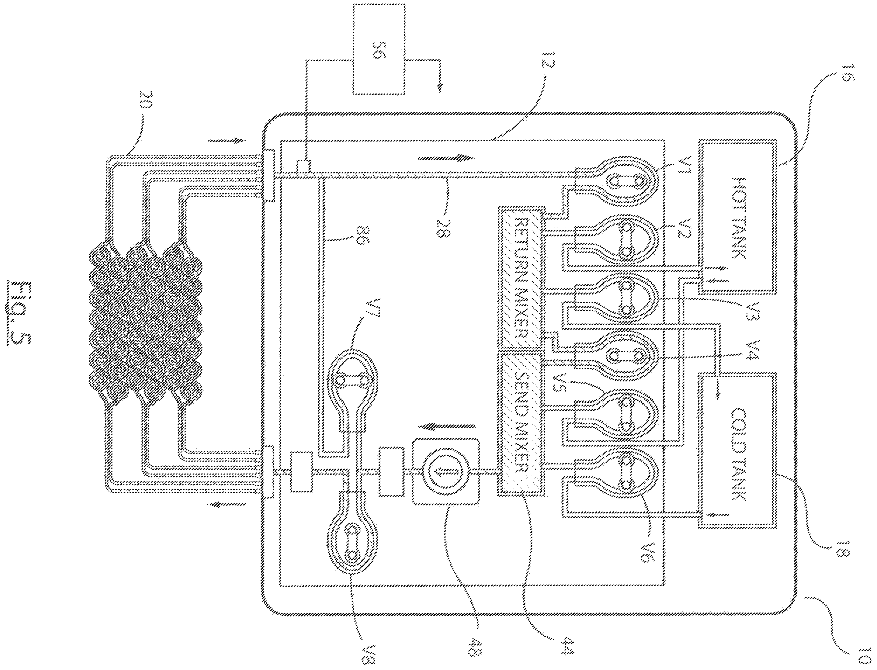

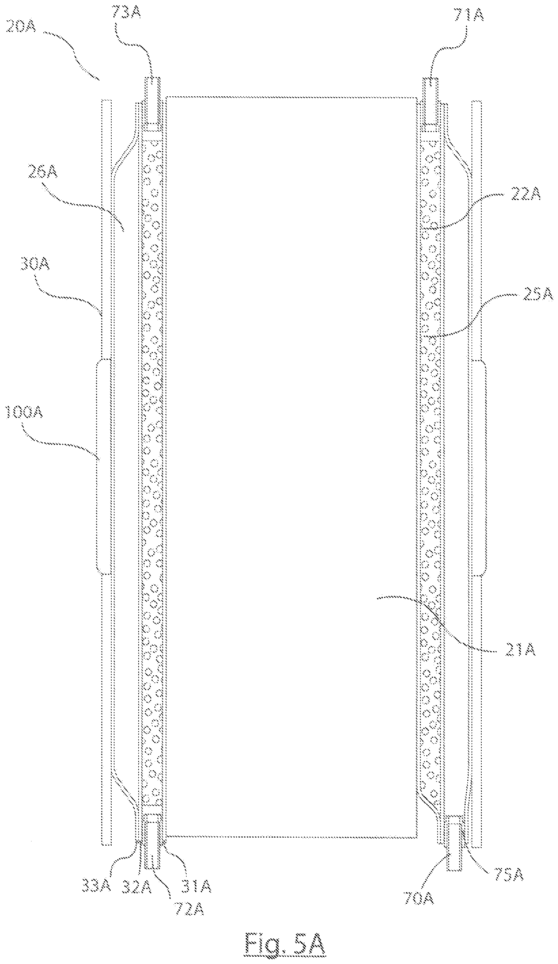

Figure 5A is a schematic diagram of an interface pack usable in embodiments of the invention in longitudinal cross-section;

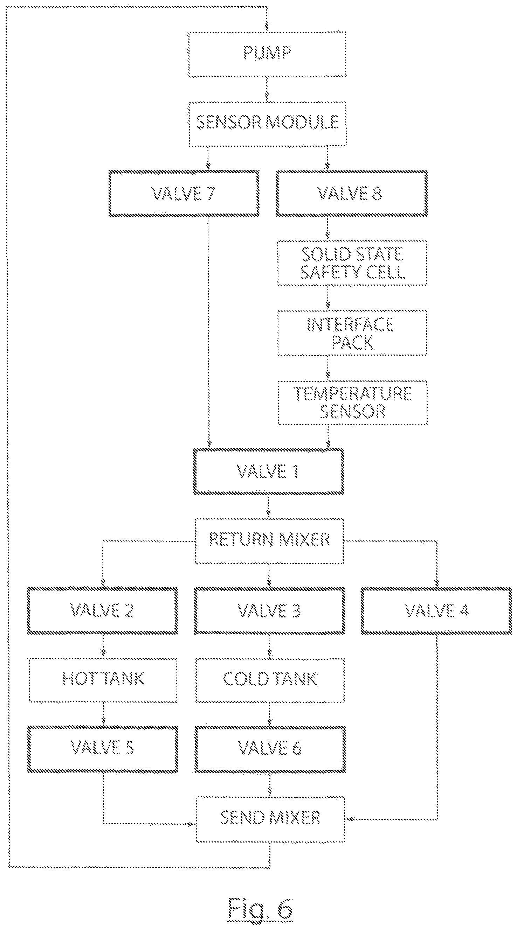

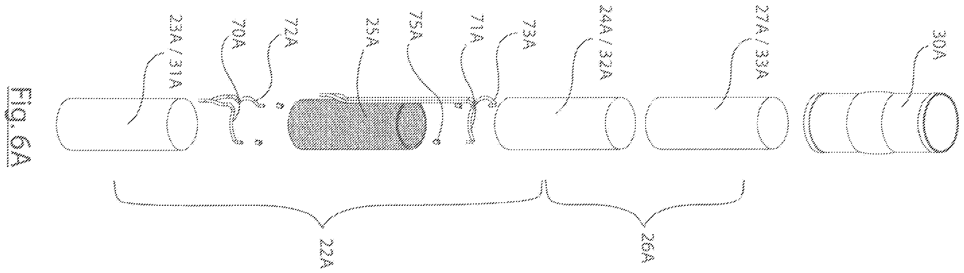

Figure 6A is an exploded schematic diagram of an interface pack usable in embodiments of the invention;

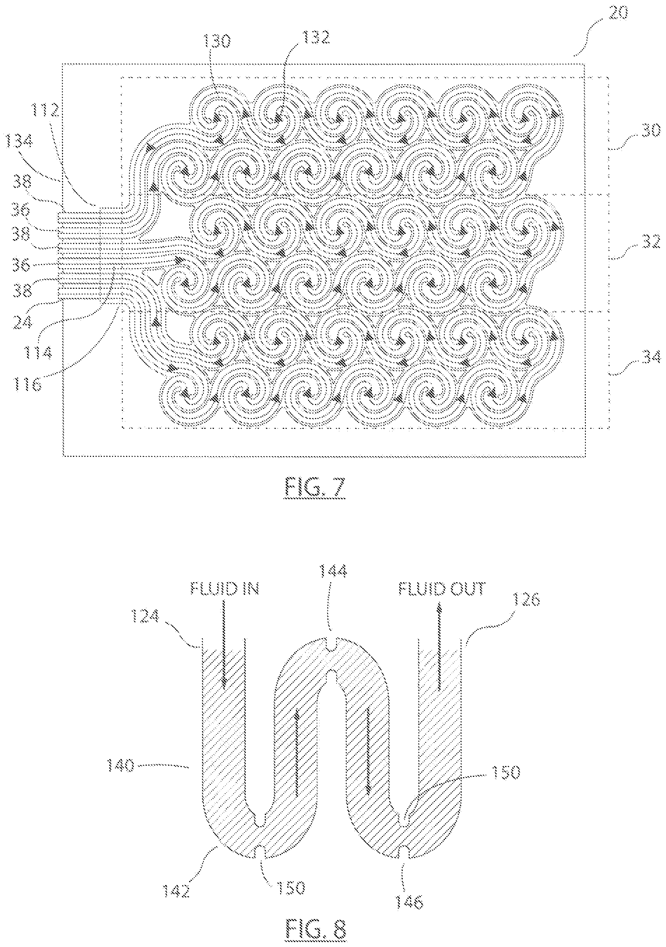

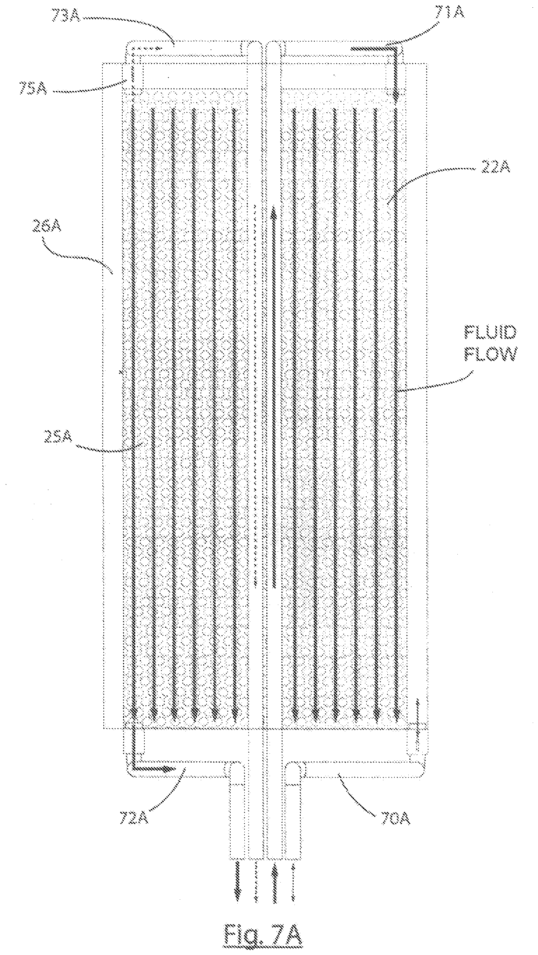

Figure 7A is a longitudinal cross-section showing the liquid feed and return tubes and the air feed tube of an interface pack usable in embodiments of the invention;

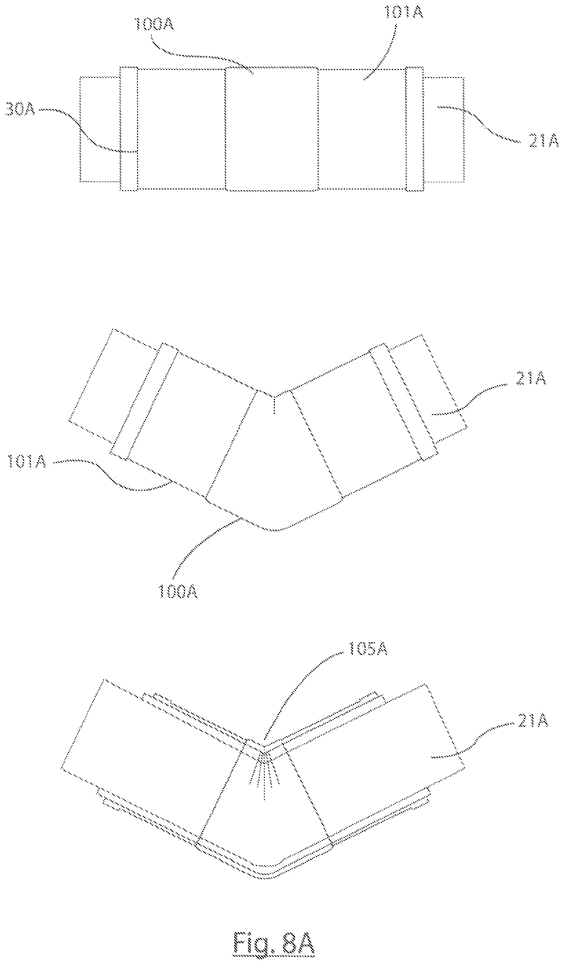

Figure 8A is a schematic diagram showing the outer jacket of an interface pack usable in embodiments of the invention;

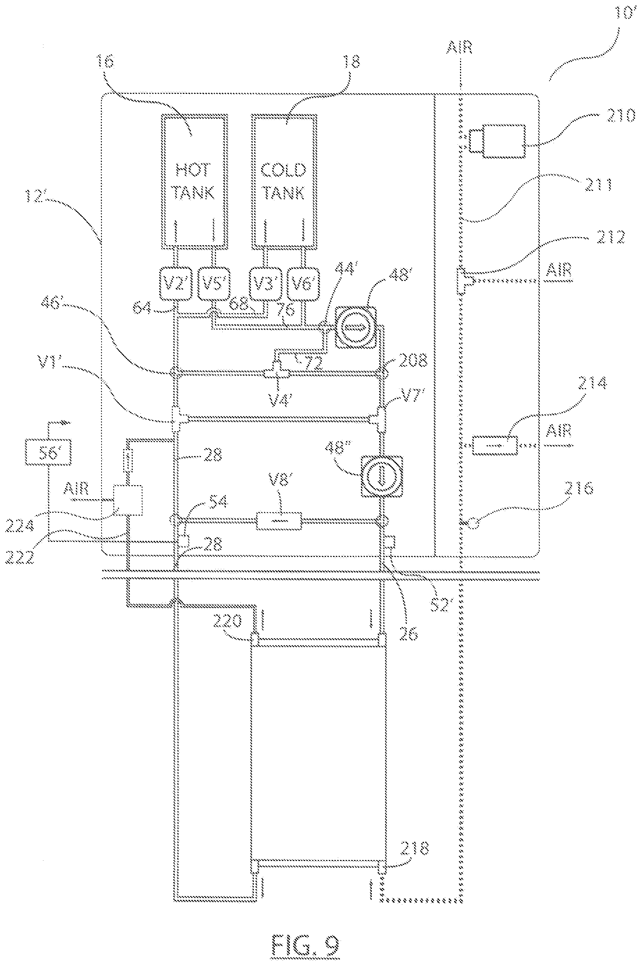

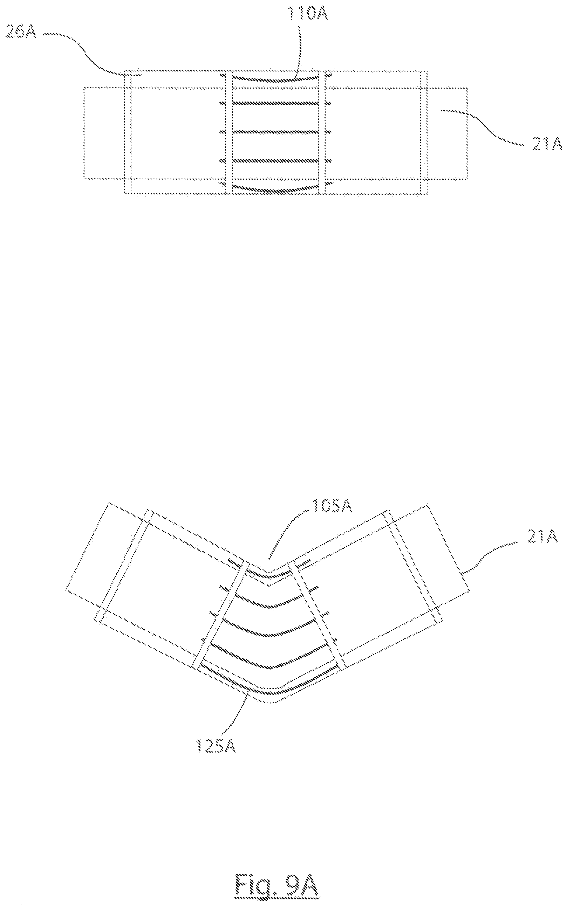

Figure 9A is a schematic diagram showing an alternative interface pack to that of Figure 8A usable in embodiments of the invention; and

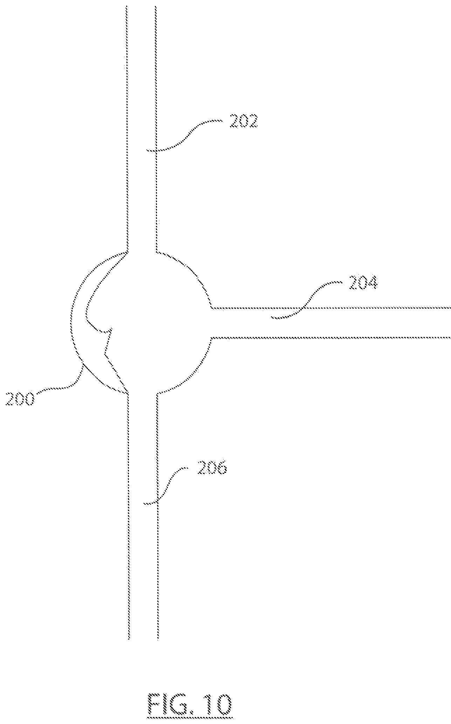

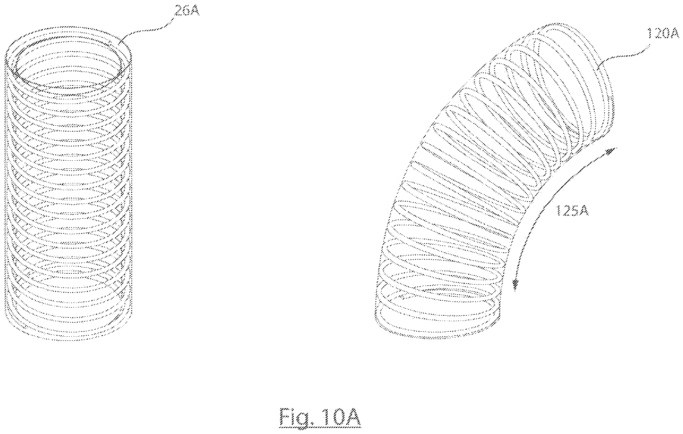

Figure 10A is a perspective view of an articulation coil for use in embodiments of the invention.

### Description of the Preferred Embodiments

Described below are various embodiments of control system and method for a thermal and pressure regulation assembly used for providing thermal and/or pressure treatment to a patient. In some modes of operation, the system and method provide cryotherapy, that is the therapeutic application of a substance to the body that provides for the withdrawal of heat from the body so as to lower tissue temperature. The use of cryotherapy following musculoskeletal injury, trauma and pre and post-surgery is common practice and has been used for many years. This type of physical therapy has several advantages over other therapeutic measures because of its limited adverse effects, low cost and easy availability. The exact physiological responses to cryotherapy are still poorly understood but the range of the body's responses to cold can be summarised as:
a) decreased metabolism;
b) reduction in abnormal body temperature;
c) effect on tissue inflammation (decreased or increased);
d) decreased muscle spasms;
e) increased tissue stiffness;
f) decreased pain;
g) effect on circulation systems (decreased or increased).

The complexity of these responses means that care must be taken to ensure that the therapy is beneficial rather than detrimental. The two main responses to effective cold treatment are a reduction in metabolism and decreased pain. By decreasing metabolism, the consumption of oxygen, cell damage and fluid build-up (swelling) are reduced. This in turn increases the recovery rate of the patient. The two main risks of incorrectly administered cold therapy are freeze burns and an increased blood flow in the injured area. The key to cold treatment is therefore the reduction and maintenance of temperature to the desired level and rate of change, at the desired depth, without damaging the skin or increasing metabolism.

Clinical studies have investigated the benefits of cryotherapy in post-operative patients following a wide range of surgeries. These studies have shown a reduction in the need for narcotic requirements, better blood drainage, and increased short term range of motion, all of which lead to a reduction in the length of hospital stay or duration of treatment.

As a consequence of the patient's improved comfort and rehabilitation there are cost benefits to be had from cryotherapy. In particular it reduces the requirement for pain relief drugs and accelerates healing and ambulation, thus reducing the recovery cycle. This can translate into considerable cost savings for hospitals and other medical institutions as patient turnover can be increased.

One study found that the reduction in time of the hospital stay for patients treated with cryotherapy was an average of 30%. Given that the cost of staying in a general or surgical ward on the NHS ranges from £400 to £1,500 per day in the case of complications, the economic benefits of an efficient cryotherapy system are clear. Cryotherapy is also firmly established in sports medicine and in the home as an effective way to treat both acute and chronic injuries as well as a broad range of common complaints. The benefits of cryotherapy when properly administered can include, amongst others:
a) accelerated healing;
b) reduction in the need for blood drainage;
c) increase in short term range of motion;
d) reduction in the length of any hospital stay;
e) reduction in the need for narcotics.

Physiotherapy clinics currently use relatively crude treatments such as ice packs or devices that provide comparatively little control. Their use can lead to skin and/or nerve damage.

The concepts disclosed herein aim to make patient recovery safer and faster by delivering cooling, warming and/or compression therapy with high levels of control and efficiency. The system and its components can also be used in the elite sports field, for recovery from cosmetic surgery and various other treatments.

Embodiments disclosed herein and in the accompanying drawings include a thermal compression engine (TCE), an integrated digital control unit and a range of disposable or reusable treatment pads or cuffs. The system works to emulate and accelerate the body's regulation of thermal energy. The therapeutic effects are achieved by precision monitoring of and intervention in the body's natural healing events via thermal and, optionally, compression therapy.

The control unit and method disclosed herein is intended to be used with a thermoregulation interface pack and a manifold assembly of the types disclosed in the applicant's earlier British patent application number GB1211149.8 and 1309310.9, and the copending PCT patent applications filed concurrently with this application under the attorney file references RJ/N30272 and RJ/N30273 and entitled "Thermoregulation Interface Pack and Assembly" and "Manifold Assembly for Thermoregulation System", respectively.

Before describing the elements and function of the control unit and thermal compression engine in detail, an example of interface pack and fluid manifold assembly is described. The skilled person will appreciate that the control unit can be arranged to operate a thermoregulation system having a different design or structure of interface pack and a different design of manifold assembly and that the examples given below are for illustration only.

Figure 1 shows an example of manifold assembly and interface pack for a thermoregulation system, in which the interface pack is designed to be conformed around a part of a patient's body such as a limb or the like. The interface pack could have any shape suitable for the particular treatment desired for a patient. For instance, in some examples it could be a sleeve or garment into which part of the patient's body to be treated can be inserted. The interface pack is intended for use with a temperature and/or pressure regulation system which can include a pump, heating and/or cooling elements for heating fluid which is then pumped via the pump through the manifold assembly into the interface pack. Such a system can provide one or more temperature sensors able to sense the temperature of fluid in the interface pack or the temperature of the interface pack itself. The sensors may be provided within the system, as part of the interface pack or as part of the manifold, or in a combination of these locations. In the embodiments described below the pump is shown as being part of the manifold assembly. It is to be understood, however, that the pump could be a separate and distinct unit.

A suitable interface pack for use with the manifold assembly taught herein is described in detail in the applicant's co-pending British patent applications GB-1211149.8 filed on 22 June 2012 and 1309310.9 and PCT patent application published as WO 2013/190333 filed concurrently herewith under the file reference RJ/N30272.

Referring to Figure 1, this shows in schematic form the principal components of an embodiment of thermoregulation system 10 which incorporates a preferred embodiment of manifold assembly 12. The thermoregulation system 10 includes a thermal supply unit 14 which includes a hot tank 16 which provides heated thermoregulation fluid, typically water, and a cold tank 18, which typically provides coolant or cold thermoregulation fluid, again typically water. The hot tank 16 and cold tank 18 could be provided with suitable heating and cooling devices of a type known in the art. These may be temperature-controllable but in the preferred embodiment this is not necessary as the hot tank 16 and cold tank 18 could be operated at individual set temperatures (hot and cold respectively), with the manifold assembly 12 designed to provide for temperature regulation of fluid supplied by the manifold. The cold tank can for example be adjustable between - 10°C and 10°C and the hot tank can be adjustable between 30°C and 60°C. The tanks can be operated at their limits or at a specific temperature within their limits.

The system 10 also includes an interface pack 20 which is coupled to the manifold 12 by suitable conduit couplings 22, 24. In the example shown in Figure 1, the manifold 12 has a single outlet conduit 26 and a single return conduit 28, which respectively feed fluid to and receive fluid from a plurality of conduits of the interface pack 20. More specifically, in this example, the interface pack 20 includes three sets 30, 32, 34 of treatment conduits for applying heat treatment from the interface pack 20. Each set 30-34 is formed of two treatment conduits, which are arranged in multiple spiral form as taught in the applicant's co-pending patent application published as WO 2013/190333 (filed under reference RJ/N30272) referred to above. The design and arrangement of the treatment conduits 30-34 and indeed of the entire interface pack 20 is not relevant to the teachings herein and described solely as an example.

Thus, the interface pack 20 includes in this example six conduits or tubes 36 at its input and, similarly, six conduits or tubes 38 at its output. The conduit coupling 22 at the outlet of the manifold assembly 12 is designed to split the supply fluid into six preferably equal channels for feeding each of the six interface pack input conduits 36. Similarly, the return conduit coupling 24 is able to combine the six fluid return paths from the interface pack outlet conduits 38 into the single return conduit 28 of the manifold assembly 12. The skilled person will readily appreciate that the conduit couplings 22, 24 can simply be in the form of fluid splitters/combiners. It is envisaged, though, that the conduit couplings 22, 24 may also include control valves for controlling whether or not the fluid is passed to any one or more of the conduits of the interface pack 20, thereby to control whether the entirety of the interface pack 20 is supplied with thermoregulation fluid or only a part thereof.

The manifold assembly 12 includes a first set of valve units 40 for controlling the mixing of thermoregulation fluid, as described in detail below, and a second set of valve units 42 for controlling the output and recirculation of thermoregulation fluid within the system 10, also described in detail below. The manifold assembly 12 also includes a supply or send mixer chamber 44 and a recirculation or return mixer chamber 46, both being coupled to the first set of valve units 40. The assembly 12 also includes a fluid pump 48, which in this embodiment forms part of the manifold assembly 12 but which in other embodiments could be a separate component provided at any suitable position within the conduit path which is able to apply pumping pressure to all the conduits of the system 10. Of course, more than one pump could be provided as appropriate.

The manifold assembly 12 also includes a sensor module 50 which is typically disposed after the pump 48 and includes a liquid temperature sensor and a flow sensor. The assembly 12 is furthermore provided with a solid state safety cell 52 which includes one or both of a temperature sensor and a pressure sensor, as well as a trigger able to cut off completely the outlet conduit 26 of the manifold assembly 12 so as to stop all fluid flow to the interface pack 20 in the case where the sensed temperature exceeds or drops below a predetermined threshold and/or when the sensed pressure exceeds a predetermined threshold.

There is also provided a temperature sensor 54 which is operable to sense the temperature of the return fluid from the interface pack 20 through the return conduit 28, used for monitoring the effect of thermal treatment through the interface pack 20. The temperature sensor 52 is coupled to a control system shown schematically at 56 and which is designed to control the operation of the system 10 to generate desired or predetermined treatment temperatures and temperature profiles within the interface pack 20. For this purpose, the control unit 56 includes an electrical connection to the temperature sensor 54, as well as electrical connections to the valve units 40, 42 and pump 48. The control unit 56 will typically be microprocessor based and a suitable structure will be apparent to the person skilled in the art.

The valve units of the sets 40, 42 may in a basic embodiment be simple on/off valves, but are preferably are of a type in which they can be fully closed, partially open or fully open and most preferably are proportional valves able to provide a plurality of partially open configurations and ideally are infinitely variable between their closed and open configurations. For example, they may be pinch or plunger valves. An example of a suitable valve unit is described below in connection with Figures 2 to 5.

The valve set 40 includes a first valve unit V1 coupled between the return conduit 28 and a first feed conduit 60 which couples into the return mixer chamber 46. A second valve unit V2 is coupled by a conduit 62 to the hot tank 16 and to the return mixer chamber 46 by a second feed conduit 64. A third valve unit V3 is coupled to an inlet conduit 66 to the cold tank 18 and to a third feed conduit 68 to the return mixer chamber 46. A fourth valve unit V4 is coupled to the return mixer chamber 46 and to the send mixer chamber 44 by conduits 70 and 72. At the supply or send mixer chamber 44, there is a fifth valve unit V5 which includes an inlet conduit 74 coupled to the hot tank 16 and a feed conduit 76 coupled to the supply or send mixer chamber 44. Finally, within this set there is a sixth valve unit V6 which has an inlet conduit 78 coupled to the cold tank 18 and a feed conduit 80 leading to the supply or send mixer chamber 44.

Thus, each of the chambers 44, 46 is coupled by an associated valve both to the hot tank 16 and to the cold tank 18, while there is a valve unit (V4) which couples the two chambers to one another and a recirculation valve unit (V1) for adjusting the amount of recirculation fluid into the fluid mix.

The supply or send mixer chamber 44 includes an outlet conduit 82 which feeds into the fluid pump 48. At the outlet of the pump 48, in this example at the outlet of the sensor module 50, there is provided a conduit T-junction 84 providing for flow of fluid from the pump 48 to the valve units V7 and V8 of the second valve set 42. Valve unit V7 is coupled to a return conduit 86 which feeds into the recirculation conduit 28, whereas the valve unit V8 couples to the outlet conduit 26 through the safety cell 52.

It is preferred that all of the valve units V1-V8 are the same as one another, and thereby are controllable by the same control parameters. It is not excluded, however, that the valves V1-V8 could be different from one another, for example, have different maximum flow capacities, closing characteristics and so on.

The valve units V1-V8, as will become apparent below, provide for complete control of the flow of fluid within the thermoregulation system 10 and in particular through the interface pack 20. Some examples of control configurations are shown in Figures 2 to 5 and described below.

As can be seen in Figures 2 to 5, the valve units V1-V8 are, in this embodiment, rotary valves having a casing providing a rounded internal wall against which there is positioned a flexible tubing or conduit 90. Disposed in the centre of each valve unit V1-V8 there is a rotor 92 having opposing rotary bearings or castors 96. The housing 88 of each valve unit V1-V8 is generally oval in plan-view, such that when the rotor 90 is aligned with the longitudinal axis of the casing 88, that is parallel to the longer dimension of the oval shape, the rotor bearings 96 are spaced from the tubing 90 of the valve unit. On the other hand, when the rotor 92 is rotated 90°, so as to be aligned with the short axis of the casing 98 of each valve unit, as shown in valve units V4 and V7 in Figure 2, the rotor bearings 96 press against the tubing 90 of the valve units, thereby to pinch the tubing closed. In this configuration, the valve units (V4 and V7) are fully closed. It will be appreciated that as the rotor 92 rotates away from this closed position, the rotor bearings 96 will gradually move away from the housing wall 88 and thereby gradually open the tubing 90 so as to allow progressively greater fluid flow through the tubing. In this manner, the valve units V1-V8 can be variably or proportionately opened. In this regard, it will be apparent the tubing 90 of the valve units V1-V8 will be resilient or elastic, such that the tubing will open as soon as pinching pressure is released.

The pump position can ensure that fluid from the return mixer is drawn into either of the tanks or straight into the send mixer via V4. Effectively, the return mixer is for fluid that has either come from the pack or been recirculated inside the manifold. The send mixer is for 'replenished' fluid from hot or cold stores and also anything that is coming straight through V4.

In Figure 2, the manifold assembly 12 has been configured by the control unit 56 to supply 50% hot fluid from hot tank 16 and 50% cold fluid from the cold tank 18. There is no recirculation of fluid within the manifold assembly 12 as the recirculation conduit 86 is closed off by the closure of valve V7. In Figure 2, the conduits which have been coupled for passage of fluid therethrough are shown shaded. As can be seen, fluid from the hot tank 16 passes into the send mixer chamber 44 by virtue of the valve units V5 and V2 being open. Similarly, fluid from the cold tank 18 also feeds into the send mixer chamber 44 by virtue of the valve units V6 and V3 being open. Fluid from the return mixer chamber 46 is split by the valve units V2 and V3 and feeds into the hot and cold tanks. The conduits leading into and out from each tank can be directional - V2, V3 and V4 only feed into the tanks or the send mixer chamber. Likewise, Valves V5 and V6 can only draw fluid from these tanks. The valve unit V4 between the return mixer chamber 46 and the send mixer chamber 44 is closed, therefore isolating the send and return mixer chambers 44, 46 from one another. Finally, the outlet valve unit V8 is open, to allow fluid flow to pass through the outlet conduit 26 into the interface pack 20.

The flow of fluid through the manifold assembly 12 is shown by the arrows in Figure 2. More specifically, fluid from the hot tank and cold tank 16, 18 flows into the send mixer chamber 44 and in practice at the same rate, therefore mixing the hot and cold fluid equally. From the send mixer chamber 44 fluid passes through the pump 48 (which is responsible for pumping fluid round the entirety of the system 10), through the outlet valve unit V8 and into the interface pack 20. Return fluid from the interface pack 20 passes through the return conduit 54 back into the return mixer chamber 28 via the first valve unit V1. The returned fluid from the return mixer chamber 46 is then fed back in equal proportions to the hot and cold tanks 16 and 18 respectively. The returned fluid from the interface pack 20 is therefore returned to the temperature regulator tanks 16, 18 to be, as appropriate, heated or cooled back to the steady state temperature of these tanks, for feeding back into the system once again. Fluid from these tanks 16, 18 then passes back into the send mixer chamber 44 via the valve units V5 and V6, again being fed in equal proportions, so as to repeat the fluid loop.

It will be appreciated that the control unit 56 may be provided with a control algorithm for ensuring that fluid is not supplied from the hot and/or cold tanks 16, 18 unless this is at the set temperature. This can usefully be achieved by providing temperature sensors at the tanks 16, 18 and by appropriate control of the valve units V2, V3, V5 and V6.

In practice, the configuration of Figure 2 is useful in providing steady state temperature within the interface pack 20, with the fluid being recirculated during this process. This configuration is particularly advantageous when there is or there is expected to be a substantial temperature differential between the outlet temperature through conduit 26 into the interface pack 20 and the return temperature from the return conduit 28. This can occur, the skilled person will appreciate, when the temperature of the fluid fed into the interface pack 20 is substantially different from body temperature at the site of treatment. This may be the case, for instance, when the system 10 is set to cool or heat a part of a patient's anatomy.

Referring now to Figure 3, this shows another configuration in which the temperature fluid to be fed into the interface pack 20 is progressively cooled. As can be seen, the valve units V2 and V5 have been closed, thereby to isolate the hot tank 16 from the fluid path. The valves to and from the cold tank 18, that is valves V3 and V6, are partially closed so as to provide only 50% fluid flow therethrough. In other words, the valves V3 and V6 are only half open. Valve units V1, V4 and V8 are fully open, whereas the recirculation valve V7 is fully closed. In this configuration, therefore, fluid will flow from the send mixer chamber 44 through the pump 48 and, via the outlet valve V8, into the interface pack 20, to return via the return path 28 and the valve unit V1 into the return mixer chamber 46. Fluid from the return mixer chamber 46 is partially fed into the cold tank 18 for cooling, via the partially open valve unit V3, whereas it is fed without restriction to the send mixer chamber 44 via the completely open valve unit V4. The proportion of fluid flow through the valves V3 and V4 is 1:2 (V3:V4).

Similarly, cold fluid supplied from the cold tank 18 passes back into the send mixer chamber 44 via the partially open valve V6 which is equally fed at a ratio of 1:2 with respect to the flow in valve unit V4. As a result, 25% of fluid reaching the send mixer chamber 44 is from the cold tank 18, whereas the remaining 75%, that is the bulk of the fluid, is received directly from the return mixer chamber 46 and thus at the return temperature from the interface pack or garment 20.

This configuration of the manifold assembly 12 is ideal for providing gentle cooling of a part of a patient's anatomy, in which only a small proportion of the fluid within the formed fluid loop is cooled at each cycle.

Referring now to Figure 4, the manifold assembly 12 is shown in a configuration in which fluid is set to recirculate internally within the manifold assembly, that is by isolation of the hot and cold tanks 16 and 18 and also of the interface pack 20. As can be seen in Figure 4, the valve units V2, V3, V5, V6 and V8 are fully closed. On the other hand, valve units V1, V4 and V7 are fully open. In this configuration, fluid is able to circulate through the send mixer chamber 44 via the recirculation conduit 86 to the return conduit 28 and back into the return mixer chamber 46 before being fed again into the send mixer chamber 44. Thus, the overall temperature of the fluid within this loop of Figure 4 is not subject to temperature changes from either of the hot or cold tanks 16 or 18 or from the interface pack 20. This configuration is particularly useful in stabilising the temperature of the conduit loop within the manifold assembly 12, which can subsequently optimise and provide fast changes in temperature within the interface pack 20. More specifically, the temperature of fluid in the loop of Figure 4 can be stabilised and then fed directly into the interface pack 20 by opening valve V8 and closing valve V7.

As an example, where the part of the patient's anatomy being treated has stabilised, the system can provide for cutting off of fluid flow within the interface pack 20 for a period, for the generation of a volume of fluid at a new temperature within the manifold 12 (which would be preceded by setting the valve units V1-V8 to provide input from one or both of the cold and hot tanks 16, 18 as appropriate before fluid isolation into the loop shown in Figure 4) with a rapid change in interface pack temperature achieved by closure of the recirculation valve V7 and opening of the outlet valve V8 to replace the fluid within the interface pack 20.

Another configuration of the manifold assembly 12 and thus of the thermoregulation system 10 is shown in Figure 5. In this configuration the hot and cold tanks 16 and 18 are isolated from the fluid loop by closure of the valve units V2, V3, V5 and V6, while fluid recirculation through the manifold only is cut off by closure of the valve unit V7. The valve units V1, V4 and V8 are open, so that fluid can pass from the send mixer chamber 44 through the pump 48, through the outlet valve unit V8 into the interface pack 20 and then from the interface pack 20 back through the return conduit 28 and valve unit V1 into the return mixer chamber 46, whereupon it can pass back into the send mixer chamber 44 via the valve unit V4. In this configuration, there is no adjustment of temperature of the fluid within the fluid circuit, apart from by heat exchange through the interface pack 20, thereby maintaining a constant temperature when the temperature of the interface pack 20 matches the temperature of the part of the patient's anatomy being treated, or for gradual change in temperature of the fluid within the circuit tending towards the patient's temperature at the site of treatment. This loop can thus provide gentle temperature stabilisation between the fluid and the patient.

It will be apparent that the manifold assembly 12 can provide numerous other configurations not shown in the examples of Figures 2 to 5. For instance, the manifold assembly 12 can also isolate the interface pack 20 from the circuit in the manifold assembly 12 and the hot and cold tanks 16 and 18, for instance in order to change the temperature within the circulation path without changing temperature of the fluid within the interface pack 20. More specifically, by closure of the valve unit V8, in a manner similar to that shown in Figure 4, fluid flow into the interface pack 20 can be stopped and with the valve unit V7 open fluid recirculated within the manifold assembly 12. Upon opening of the valve units V2, V3, V5 and V6, as appropriate, the temperature of fluid within the recirculation path can be changed to a desired temperature, which can then be fed into the interface pack 20 by closing the recirculation valve unit V7 and opening the outlet valve unit V8, so as to provide a new volume of fluid into the interface pack 20 at the new temperature, generating a very rapid temperature change within the interface pack 20 and rapid provision of a new treatment temperature. This can provide for very rapid changes in treatment temperature, which can be particularly useful for some treatments.

The pump unit 48 may operate in a single flow direction as indicated by the arrows in the Figures. Other embodiments may provide for the pump unit 48 to be able to pump in both directions, which can be useful in creating pressure differentials within the interface pack 20. However, pressure differentials can also be created by selective operation of the pump unit 48 and/or by opening and closing as appropriate the valve units V7 and V8. Specifically, as valve unit V8 is opened and closed, this will change the fluid pressure within the interface pack 20. The valve unit V8 can thus be operated to create pressure pulses within the interface pack 20. When the valve unit V8 is closed, the valve unit V7 should be opened (but not necessarily in all cases), in order to provide a continuous path for fluid within the circuit. Of course, as an alternative, the pump could be selectively activated and deactivated in the appropriate sequence, while in other cases, dependent upon the speed of opening and closing of the valve unit V8, it may not be necessary to make any other changes to the operation of the pump or other valve units V1-V7.

Figure 6 is a flow diagram useful in depicting the interrelationship between the various elements of the system 10 and the valve units of the manifold assembly 12. With reference to Figure 6, the pump unit 48 provides the fluid pressure to generate fluid flow within the various configurable fluid circuits of the manifold assembly 12 and thermoregulation system 10. The sensor module 50 is disposed immediately downstream of the pump 48 and thus able to sense parameters such as fluid temperature and fluid flow rate. Fluid from the pump 48 can pass selectively through the valve unit V7 or valve unit V8, in dependence on their configuration. In the case of recirculation, fluid passes via the valve unit V7 directly back into valve unit V1. When fluid is to be output from the manifold assembly 12, this passes through valve unit V8, through the solid state safety cell 52 (which will close off all fluid flow in the case of the sensing of an abnormality in the parameters of the fluid), into and through the interface pack 20 back into the manifold assembly 12 via the return temperature sensor 54. As explained above, the sensor 54 senses the return temperature of the fluid from the interface pack 20, useful in determining the efficacy of the thermal treatment. Fluid then flows back to valve unit V1, which passes the fluid into the return mixer chamber 46. Fluid from the return mixer chamber 46 can pass, selectively, into one or more of valve units V2, V3 and V4 and as appropriate, into the hot and/or cold tanks 16 and 18 and from the latter selectively back into the send mixer chamber 44 via valve units V5 and V6. If any of the fluid is to bypass the hot and cold tanks 16, 18, this will pass via valve unit V4 directly into the send mixer chamber 44, which then feeds the fluid back into the pump 48 and thus back into the fluid circuit.

It will be apparent from the above that the manifold assembly 12 provides a configurable fluid circuit system which is able to provide many different configurations of fluid circuit and as a result a significantly more sophisticated thermoregulation system than is possible in the prior art.

In addition to the configurations described above, it will be appreciated that the proportional adjustment aspect of the valve units V1-V8 enables them to be partially opened or closed, thereby to allow partial and a proportion of fluid to flow through the valve units and their associated conduits, in practice providing an infinitely variable configuration of fluid paths and temperature control to the thermoregulation system 10. The control can thus be significantly more sophisticated than prior art systems which either provide for mixing of fluids within the interface pack 20 itself or by simple open/closed valves.

Referring now to Figure 7, this shows in detail an example of the interface pack 20 in schematic form. The interface pack may be a substantially planar structure, preferably flexible to be conformable to a patient, and may have other forms as well, such as a cuff or garment part. The interface pack 20 is formed of two layers of impermeable material, advantageously a polymer, which are bonded together to form therewithin a plurality of conduits 112, 114, 116, each of which extends in a respective temperature regulation treatment zone 30, 32, 34. Each conduit 112-116 includes a respective feed path 36 and return path 38. It will be appreciated that the number of conduits 112-116 and the number of treatment zones 30-34 may vary with, in some cases, there being only a single conduit and treatment zone, while in other cases there may be provided two or more than three sets of conduits and treatments zones. Moreover, the treatment zones 30-34 may not necessarily be aligned side-by-side as in the example of Figure 7, as these could be arranged in an array or any other manner desired or optimal for a particular application.

The conduits 112-116, and as a result the treatment zones 30-34, in this embodiment extend over substantially entire surface area of the interface pack 20. In other embodiments, the temperature regulation treatment zones 30-34 may extend only over a part of the interface pack 20, for instance but not necessarily in a central portion thereof.

With respect to the example shown in Figure 7, each conduit 112-116 is arranged in a plurality of spirals 130. In this example six spirals 130 are disposed in what could be termed the feed direction and six spirals disposed in what could be termed the return direction of the conduit, for a total of twelve spirals to each conduit 112-116. Again, the number of spirals 130 would be dependent upon the size of the interface pack 20 in particular of the heat treatment zones.

Each spiral 130 provides a forward and a return path therein and a path inversion at point 132, located at the centre-point of the spiral. Thus, in this example, each conduit or fluid path 112-116 provides twelve path inversions along its length. The advantages of these is described in detail below.

As will be apparent from Figure 7, the conduits 112-116 are all aligned at a common coupling zone 134 of the interface pack 20, for coupling to a suitable fluid source. With this embodiment, thus, the fluid source would typically include a suitable manifold providing three fluid inlets and three fluid return paths. These may be commonly connected so as to provide equivalent fluid flows through the conduits 112-116 but in other embodiments may be separately fed so as to provide different fluid flows through each of the conduits and thus in each of the temperature regulation treatment zones 30-34.

Referring now to Figure 8, there is shown another embodiment of conduit 140 having similarities to the conduits 112-116. However, instead of having spiral paths 130, the conduit 140 has what could be described as a zigzag shape, with a plurality of path inversions 142-146. It will be appreciated that in practice the conduit of the example of Figure 8 would have many more points of inversion 142-146 than shown in Figure 8, which is to be taken as schematic only.

Figure 8 also shows an example of flow constrictor 150, which may also be provided in the conduits 112-116 of the example of Figure 7. The flow constrictors are located at the points of path inversion. As shown in Figure 8, the flow constrictors 150 may be what can be described as pinched zones of conduit wall, which provide a restriction to the path of fluid through the conduit. In other examples, the flow restrictors 150 could be baffles within the conduit. The purpose of the flow restrictors 150 is to generate turbulence at the point of path inversion. This turbulence ensures that there is no stagnation of fluid at the point of path inversion, which could otherwise lead to the generation of laminar flow, which would contribute to loss of thermal transfer efficiency of the interface pack.

Of course, the flow constrictors could have any other suitable form and structure.

Another embodiment of a thermoregulation system 10' including a manifold 12' is shown in Figure 9. This embodiment operates in a similar manner to the embodiment of Figure 1, except as outlined below.

The description below refers to thermoregulation fluid and pressure fluid. Where the term "fluid" is used without being stipulated as thermoregulation fluid or pressure fluid then, unless the context clearly dictates otherwise, the term is referring to thermoregulation fluid.

In the embodiment of Figure 9, at each conduit junction there is provided a mixing chamber 200 such as shown in Figure 10. As shown in Figure 10, a conduit junction will typically have three branches coupled to the mixing chamber, either two input branches and one output branch, or one input branch and two output branches.

In Figure 10, the two branches 202, 206 that are the same (input or output) are shown as being opposite with respect to the mixing chamber 200. The precise arrangement of branches is not essential. However, where two branches are input branches, these branches are preferably arranged so that their fluid inputs into the mixing chamber are separated by between 90° and 270°, preferably by about 180°. This causes most efficient turbulence and mixing of the fluid flows.

In Figure 10, the mixing chamber is cylindrical in shape, being circular in the plane of fluid flow. However, any shape that is wider than any input branch and provides space for turbulence and mixing can be used.

In the embodiment of Figure 9, valve units V2', V3', V5' and V6' serve a purpose corresponding to the valve units V2, V3, V5 and V6 of Figure 1. However, valve units V2', V3', V5' and V6' in the embodiment of Figure 9 are electronic valve units which can be opened and closed at high frequency by a control unit 56'.

Owing to the use of mixing chambers at conduit junctions in the embodiment of Figure 9, the dedicated send and return mixers 44, 46 of Figure 1 are not needed.

In the embodiment of Figure 9, there is a first mixing chamber 200 at a junction of conduit 80 from an output of valve unit V6' and conduit 76 from an output of valve unit V5', and a send mixer 44' is provided by a second mixing chamber at a junction of a conduit from an output of the first mixing chamber and conduit 72 from an output of valve unit V4'.

A dosing pump 48' is coupled by a conduit to an output of the send mixer 44'. The dosing pump 48' can be similar to the pump 48 described above. An output of the dosing pump 48' is coupled by a conduit to a conduit junction 208.

There is a third mixing chamber 200 at a junction of conduit 64 coupled to an input of valve unit V2' and conduit 68 coupled to an input of valve unit V3'. A return mixer 46' is provided by a fourth mixing chamber at a junction of a conduit to the third mixing chamber and conduit from an output of valve unit V1'.

Valve units V1', V4' and V7' are electronic three-way valve units which can also be opened and closed at high frequency by the control unit 56'. Valve unit V1' is coupled between the return conduit 28 and a conduit coupled to the return mixer 46'. Valve unit V1' is also coupled via a conduit to valve unit V7'.

Valve unit V7' is coupled between a conduit coupled to the conduit junction 208 and a conduit coupled to a circulation pump 48". The circulation pump 48" can be similar to the pump 48 described above. As described above, valve unit V7' is also coupled via a conduit to valve unit V1'.

Valve unit V4' is coupled between a conduit coupled to the return mixer 46' and a conduit coupled to the conduit junction 208. Valve unit V4' is also coupled to a conduit coupled to the send mixer 44'.

An output conduit of the circulation pump 48" is coupled via a sensor 52' to an outlet conduit 26 of the manifold 12'. The sensor 52' can include a temperature sensor, a pressure sensor and a flow rate sensor.

The outlet conduit 26 can be configured as described above for coupling to an interface pack such as described above. Additionally or alternatively, the outlet conduit can be coupled to a tubular cuff such as described in British patent application number GB 1309310.9, and such as described below in connection with Figures 1A to 10A.

Valve unit V8' is coupled between the output conduit of the circulation pump 48" and the return conduit 28. The valve unit V8' is a mechanically operated overpressure valve. It allows fluid to pass through it only in one direction, in this case from the pump 48" to the return conduit, and only when the fluid pressure exceeds a threshold pressure. This means that if the pressure at the output of the circulation pump 48" gets too great, the valve unit V8' will allow some of it to pass straight to the return conduit 28, preventing too much pressure being put into the interface pack.

The valve unit V1' (or V1 in the embodiment of Figure 1) can be controlled to affect the pressure of the thermoregulation fluid in the interface pack by restricting or allowing the return of fluid from the interface pack. The pressure of the thermoregulation fluid in the interface pack can also be controlled by the pumps and by other valves which can affect a flow rate of thermoregulation fluid into the interface pack.

The embodiment of Figure 9 also includes a pressure system. The pressure system includes a pump 210 operable to pump a pressure fluid from a pressure fluid source through an output conduit 211. Typically, the pressure fluid is air and the pressure fluid source is the atmosphere. However, the pressure fluid can serve also as thermoregulation fluid, for example by being temperature controlled air, thereby avoiding the need for separate pressure and thermoregulation fluid flows.

The output conduit 211 is coupled to a three-way valve 212 which is operable selectively and variably to divert pressure fluid from the pump 210 to a pressure fluid sink, typically the atmosphere, or to allow it to continue in the output conduit 211 towards the interface pack. This enables the pressure of the pressure fluid to be varied. However, the pump can in some embodiments also or alternatively be controlled to vary the pressure of the pressure fluid.

A mechanically operated overpressure valve 214 is coupled to the output conduit 211 which allows fluid to pass from the output conduit 211 to the pressure fluid sink only when the pressure in the output conduit 211 exceeds a threshold value. The overpressure valve 214 does not allow fluid to pass from the sink to the output conduit 211. The overpressure valve allows the release of excess pressure to ensure that too much pressure is not provided to the interface pack.

A pressure sensor 216 is provided which is operable to measure the pressure of fluid being provided to the interface pack. The pump 210, value 214 and pressure sensor 216 are electrically coupled to and thereby controllable by the control unit 56'.

The output conduit 211 is coupled to a pressure fluid inlet 218 of the interface pack, exemplary operation of which is detailed below with respect to Figures 1A to 10A.

As described in more detail below, an interface pack may include a liquid restricted return 220 which is coupled to an input conduit 222. The input conduit 222 is coupled via a separator valve 224 to the return conduit 28. The separator valve is configured to separate pressure fluid from thermoregulation fluid in the input conduit 222. Typically, the pressure fluid is air and the thermoregulation fluid is a liquid such as water, and the separator valve 224 allows air to escape to the atmosphere.

A valve can be provided to variably restrict the return of pressure fluid to the thermoregulation assembly. This function can be provided by separator valve 224 or by a separate valve in the manifold assembly or in the interface pack. Variable operation of this valve can affect the amount of pressure fluid allowed to return and thereby affect the pressure of the pressure fluid in the interface pack.

The pressure system can be used to provide a desired compression treatment to a user. As described below, it can also be used to vary a characteristic energy transfer rate between an interface pack and a user. The pressure can be controlled for example to increase or decrease the pressure or to pulse the pressure.

The pressure system can in some embodiments be used in combination with the system of the embodiment of Figure 1.

As with the embodiment of Figure 1, the embodiment of Figure 9 can be used to provide a variety of functions. In some embodiments, the system can be used to provide an interface pack within the range of -5°C to 45°C with or without compression from the pressure system. The operation and configuration of the system is controlled by the control unit 56'.

In order to achieve a 50:50 mix of hot and cold thermoregulation fluid in a manner corresponding to the arrangement of Figure 2, valve unit V1' is configured to direct fluid from the return conduit 28 to the return mixer 46' and not to the valve unit V7'. Likewise, valve unit V7' is configured to direct fluid from the chamber 208 to the circulation pump 48" and not to valve unit V1'.

The control unit 56' operates each of valve units V2', V3', V5' and V6' to repeatedly open and close at high frequency. Valve units V2' and V5' are open while valve units V3' and V6' are closed to allow fluid from the return mixer 46' to pass to the hot tank 16 and fluid from the hot tank to pass to the send mixer 44', and valve units V3' and V6' are open while valve units V2' and V5' are closed to allow fluid from the return mixer 46' to pass to the cold tank 18 and fluid from the cold tank to pass to the send mixer 44'. In this example, the control unit 56' is configured so that each of valve units V2', V3', V5' and V6' are open for equal lengths of time, thereby circulating fluid from the hot and cold tanks substantially equally, creating a substantially equal mix of hot and cold fluid in the system. The valve unit V4' is closed, thereby isolating the return mixer 46' from the send mixer 44'.

Fluid from the send mixer 44' is pumped by the dosing pump 48' through chamber 208 and to circulation pump 48". Valve unit V7' is configured so that fluid can only flow towards pump 48" and not towards valve unit V1'. Circulation pump 48" pumps fluid into the interface pack via the sensor 52'.

Fluid returned from the interface pack passes temperature sensor 54 and through return conduit 28 to return mixer 46'.

In order to achieve a progressive cooling in a manner corresponding to the arrangement of Figure 3, the control unit 56' operates the valve unit V4' to repeatedly change at high frequency so that 75% of the time it allows fluid to pass from the return mixer 46' to the send mixer 44' and 25% of the time it blocks fluid received from the return mixer 46'. Valve units V2' and V5' are closed to prevent fluid passing to or from the hot tank. Valve units V3' and V6' are opened and closed at high frequency so that they are open when valve unit V4' is blocking fluid received from the return mixer 46' and closed when valve unit V4' is directing fluid from the return mixer 46' to the send mixer 44'. In this way, returned fluid at the return mixer 46' is passed 75% of the time directly to the send mixer 44' and 25% of the time to the cold tank, and fluid at the send mixer 44' is received 75% of the time from the return mixer 46' and 25% of the time from the cold tank before being circulated by pumps 48' and 48".

An advantage of the embodiment of Figure 9 is that functions corresponding to those of Figures 4 and 5 can be performed simultaneously.

Valve unit V1' can be configured to direct all fluid returning from the interface pack towards valve unit V7' and valve unit V7' can be configured to block all fluid arriving from dosing pump 48' and to direct all fluid received from valve unit V1' to circulation pump 48". In this way, the system is divided into two isolated circuits.

Fluid returning from the interface pack is routed from valve unit V1' to valve unit V7' and then pumped back into the interface pack by circulation pump 48". Although the fluid does not reach return mixer 46' or send mixer 44', fluid is recirculated around the interface pack without being warmed or cooled by the hot and cold tanks, in a manner corresponding to Figure 5.

At the same time, valve units V2', V3', V5' and V6' are closed, isolating the hot and cold tanks. Valve unit V4' is configured to direct fluid from chamber 208 to the send mixer 44', meaning that fluid is circulated from the dosing pump 48' to the chamber 208, to the valve unit V4' and back to the dosing pump 48'.

However, the advantages of being able to provide separate circuits can be particularly seen where the hot and/or cold tanks are used and the valve unit V4' allows some fluid to pass from the chamber 208 to the return mixer 46'. In this way, fluid circulated by pump 48' can be brought to a desired temperature in isolation of the interface pack before being applied to the interface pack, but while allowing the interface pack to continue operating with its existing fluid.

In effect, in the embodiment of Figure 9, the control unit 56' is configured to operate the manifold 12' in four modes of operation:
1. Circulation in isolation of the hot and cold tanks;
2. Circulation in isolation of the interface pack;
3. Circulation exclusively via the hot tank; and
4. Circulation exclusively via the cold tank.

As can be seen from the above description, there may be more than one possible circuit for each mode. For example, mode 1 can include: circulation by the dosing pump 48' via the chamber 208 and the valve unit V4' and/or circulation by the circulation pump via the interface pack and valves V1' and V7' and/or circulation by the dosing and circulation pumps via the interface pack, valve unit V1', return mixer 46', valve unit V4', and send mixer 44'.

Furthermore, modes 3 and 4 can be operated simultaneously with or separately from mode 2 depending on whether fluid is being brought to temperature in isolation of the interface pack or circulated via the interface pack from the hot or cold tanks. An important point to note is that for modes 3 and 4, all circulating thermoregulation fluid passes through the hot or cold tank respectively. The control unit 56' is operable to interchange between modes at high frequency in order to provide a desired operation. In particular, the system is configured so thermoregulation fluid is not circulated via the hot tank at the same time as via the cold tank. The control unit is configured to operate one or more of the valve units at high frequency to interchange between circulating via the hot tank via the cold tank or via neither in order to achieve a desired temperature. For example, as described above, for obtaining a 50:50 mix of hot and cold fluid, the control unit 56' is operated to interchange between modes 3 and 4 at high frequency, where modes 3 and 4 are operated without mode 2. In the example of progressive cooling described above, mode 4 without mode 2 is operated 25% of the time, and mode 1 (circulation by the dosing and circulation pumps via the interface pack, valve unit V1', return mixer 46', valve unitV4', and send mixer 44') 75% of the time.

An embodiment of a control system for a thermoregulation assembly is described below with reference to the embodiment of the thermoregulation system shown in Figure 9. However, the control system can be used, subject to appropriate modifications, with any thermoregulation regulation assembly described herein.

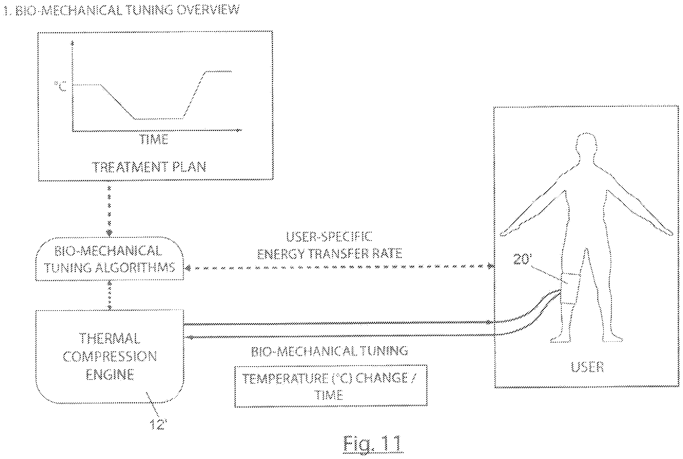

As shown in Figure 11, an interface pack 20' is placed around a part of a user's body, in this case his leg.

The user has a treatment profile which includes a desired temperature profile 226. The desired temperature profile is a profile of desired temperatures for the interface pack 20' over time. The treatment profile may also include a profile of other parameters such as pressure.

However, it is not always appropriate to supply thermoregulation fluid to the interface pack 20' at the desired temperature according to the desired temperature profile. Reasons for this include that the temperature of the supplied thermoregulation fluid does not necessarily equal the temperature of the interface pack 20' since there can be heating and cooling effects from the surroundings and the user. In addition, if temperatures are supplied which are too hot or too cold for a particular user, they can cause too much energy transfer, potentially damaging tissue, or too little, which may prevent the system from being effective.

Different users respond differently to the application of heat or cold, depending on their resistance to energy transfer.

The control system is operable to vary a plurality of feed parameters which can affect the amount of energy passing through the interface pack. These parameters include the temperature of the thermoregulation fluid being supplied to the interface pack, the volume of thermoregulation fluid in the interface pack, the rate of flow of thermoregulation fluid to the interface pack, the pressure of the thermoregulation fluid, and, in embodiments which utilise the pressure system, the pressure of the pressure fluid applied to the interface pack. However, it is advantageous to keep the pressure of the thermoregulation fluid greater than the pressure of the pressure fluid to prevent thermoregulation fluid becoming trapped in the interface pack. The feed parameters can be varied by the control unit 56' by adjustment of the pumps and valves described above.

In order to determine the appropriate feed parameters to comply with the desired temperature profile for a particular user, the control unit 56' is operable to obtain a conversion algorithm.

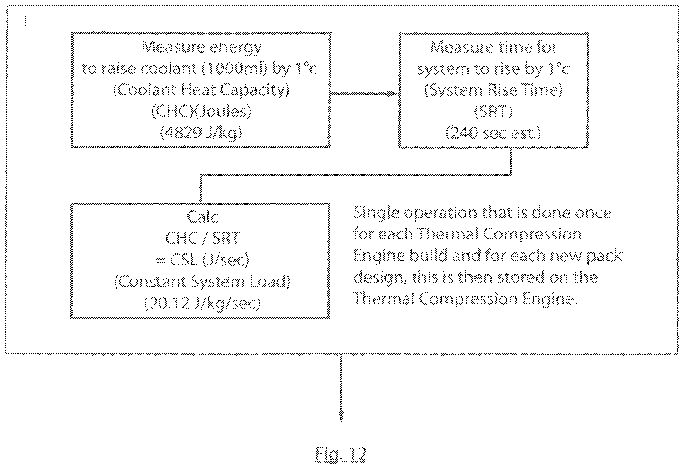

The system is calibrated. This is generally performed once when a new system is prepared and before it is supplied to a user. A flow chart of this process is shown in Figure 12.

First, the specific heat capacity (CHC) of the thermoregulation fluid is determined.

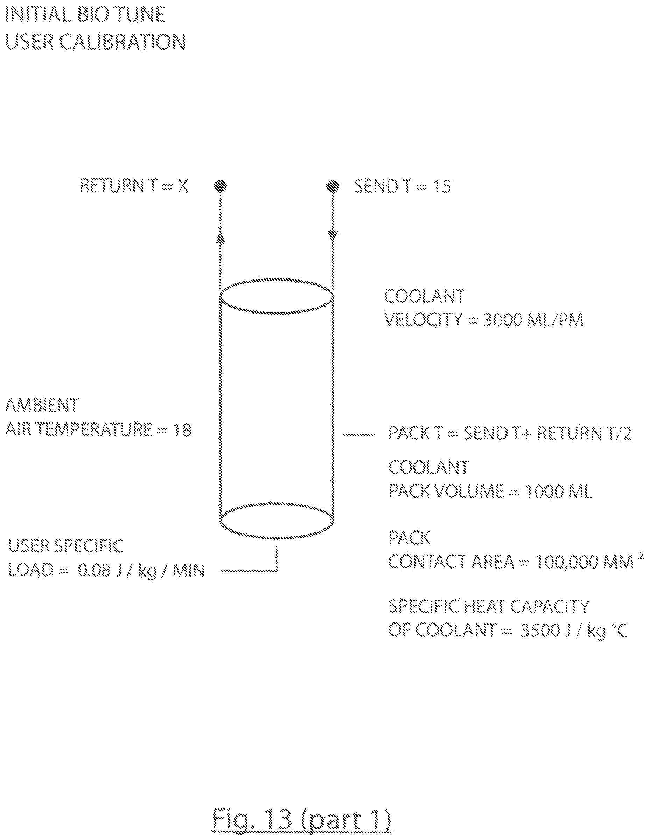

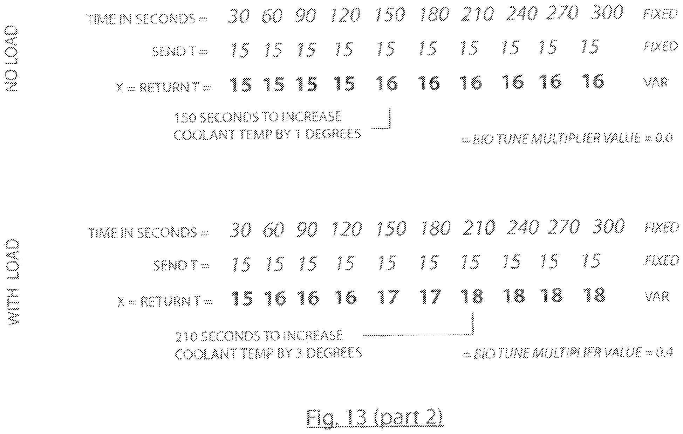

Then, as shown in Figure 13, with the interface pack free of a load, that is without being placed on a user, the thermoregulation system is operated with fixed predetermined feed parameters for a fixed length of time. During this time, the sensor 52' measures the temperature, flow rate and pressure of thermoregulation fluid being supplied to the interface pack, and, where appropriate, the pressure sensor 216 measures the pressure of the pressure fluid being supplied. The temperature sensor 54 measures the temperature of the returned thermoregulation fluid from the interface pack. These data are supplied to the control unit 56'.

The control unit 56' calculates the difference between the temperature measured by sensor 52' and the temperature measured by temperature sensor 54. The temperature of the interface pack is calculated as the average of these temperatures. From these data, the rate of change of the temperature of the interface pack is calculated. A characteristic energy transfer rate of the unloaded pack (CSL) is calculated by multiplying the specific heat capacity of the fluid by the rate of change of the temperature of the interface pack. This is then stored by the control unit for example in a local data memory.

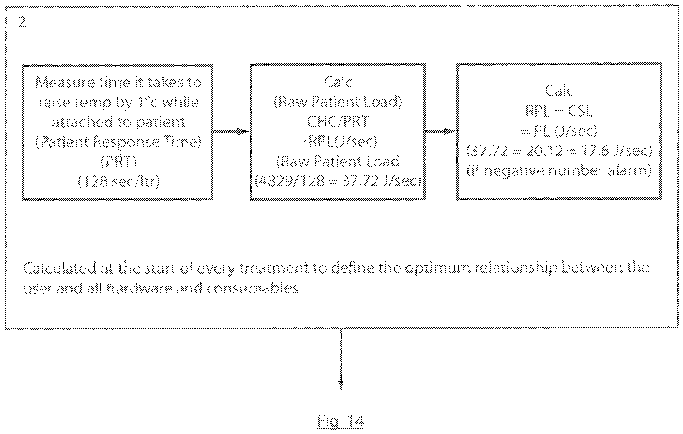

Once a system is supplied to a user but before a treatment plan is commenced, a characteristic energy transfer rate between the user and the interface pack is calculated with the interface pack placed around a user. A flow chart of this process is shown in Figure 14.

As per the calculation of the characteristic energy transfer rate of the unloaded pack, the thermoregulation system is operated with fixed predetermined feed parameters for a fixed length of time and data is measured and supplied to the control unit 56'. From these data, the rate of change of the temperature of the interface pack is calculated. This can be by measuring how long it takes to raise the fixed volume of fluid by 3 or 4 degrees. An absolute energy transfer rate of the loaded pack (RPL) is calculated by multiplying the specific heat capacity of the fluid by the rate of change of the temperature of the interface pack. The characteristic energy transfer rate between the interface pack and the user is determined by calculating the difference between the absolute energy transfer rate of the loaded pack and the characteristic energy transfer rate of the unloaded pack.

As will be apparent, the characteristic energy rates above are specific energy rates. In other words they are per unit mass.

In other words, the characteristic energy transfer rate between the user and the interface pack provides the amount more (or less) specific power that needs to be provided to the interface pack as compared with a system with no load in order most efficiently to cause a change in temperature.

As will be apparent to the skilled person, the specific power provided to the interface pack can be varied by varying the mass of fluid in the interface pack, by varying the difference in temperature between the fluid being supplied to the interface pack and the interface pack temperature, and/or by varying the rate of flow of interface pack fluid.

Furthermore, the skilled person will appreciate that the characteristic energy transfer rate between the user and the interface pack will be affected by the pressure of the thermoregulation fluid, a pack contact area, and the pressure of the pressure fluid, if any.

The control unit 56' is operable to obtain a conversion algorithm dependent upon the characteristic energy transfer rate between the user and the interface pack. The conversion algorithm provides an algorithm for converting a desired interface pack temperature into one or more feed parameters which enable the system to bring the interface pack to or maintain the interface pack at that desired temperature. In a preferred example, the control unit 56' is operable to access a remote or local look-up table or graph which provides a conversion algorithm for a given characteristic energy transfer rate.

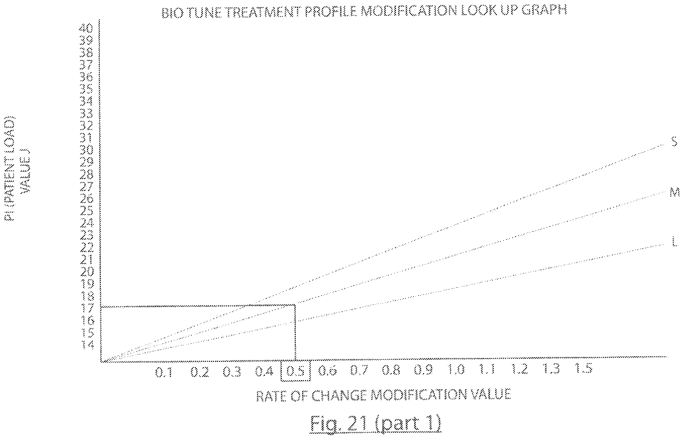

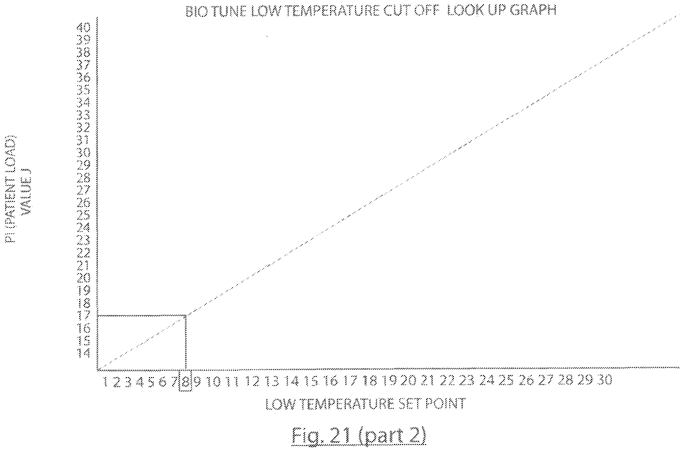

Figure 21 shows an example of such look-up graphs, one for obtaining a low temperature limit and one for obtaining a rate of change for applying to a desired temperature profile. Both graphs are dependent upon a characteristic energy transfer rate.

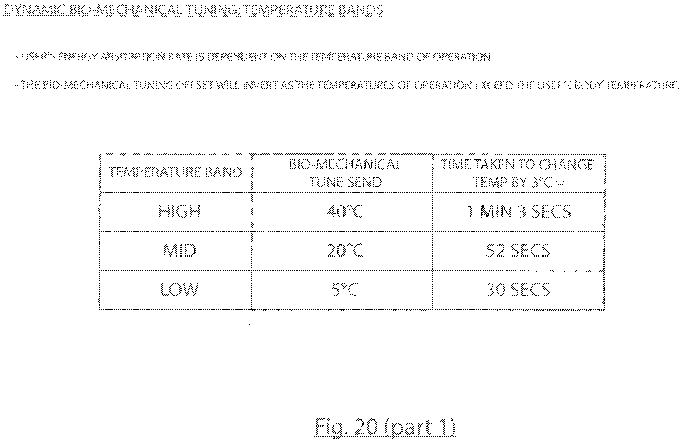

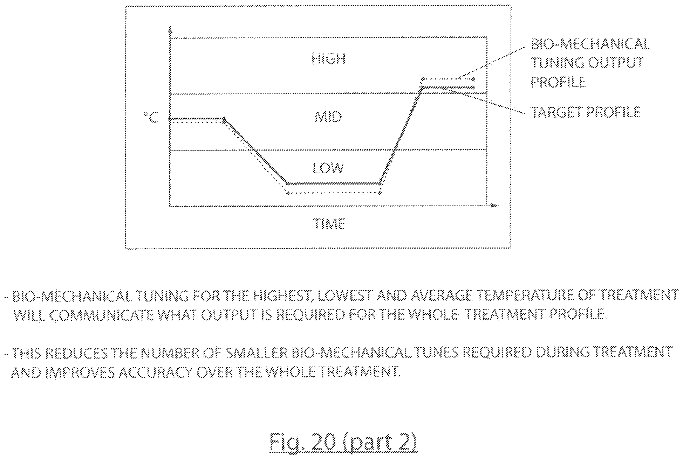

In some embodiments, the control unit 56' is operable to obtain a conversion algorithm for each of a plurality of temperature bands. An example of this is shown in Figure 20. In order to do this, the control unit 56' is operable to determine a characteristic energy transfer rate between the user and the interface pack of each of a plurality of temperatures. Typically, a normal range of operation of the pack is divided into a plurality of temperature bands, for example a high, mid and low temperature band. In order to calculate a conversion algorithm for each of the temperature bands, the method described above for calculating a characteristic energy transfer rate between the user and the interface pack is performed with the fixed temperature of thermoregulation fluid being a temperature characteristic of the band. For example, for a high band, the characteristic temperature may be 40°C, for a mid band, the characteristic temperature may be 20°C, and for a low band, the characteristic temperature may be 5°C. The control unit 56' is then operable to obtain a conversion algorithm for each of the bands dependent on the characteristic energy transfer rate for that band.

Advantageously, the process of calculation of the characteristic energy transfer rate between the user and the interface pack also brings the interface pack to a predetermined starting temperature. Since the energy available to be lost or absorbed by the interface pack is dependent, amongst other factors, on the difference in temperature between the fluid being supplied to the interface pack and the interface pack temperature, it is advantageous if the starting temperature of the interface pack is known when the conversion algorithms are derived. By using the calculation of the characteristic energy transfer rate to bring the interface pack temperature to a predetermined starting temperature, the conversion algorithms available to the control unit 56' can be provided on the basis of the interface pack temperature at the beginning of a treatment method being the predetermined starting temperature.

As described above, the characteristic energy transfer rate is dependent upon a number of feed parameters and the specific power provided to the interface pack is also dependent upon a number of feed parameters. The control unit 56' may be operable to obtain a conversion algorithm which maintains a plurality of feed parameters at fixed values and provides one or more formulae for varying others. Typically, the volume or mass of fluid in the interface pack is held at a fixed value. Furthermore, factors such as the pack contact area and the specific heat capacity of the thermoregulation fluid can be useful for calculating a characteristic energy transfer rate but in practice are typically held constant.

As described above, the pressures of the thermoregulation fluid and the pressure fluid are governed by the advantages of the pressure of the thermoregulation fluid being greater than the pressure of the pressure fluid. However, the pressure of the thermoregulation fluid and/or the pressure of the pressure fluid can varied in order to vary the compression of the interface pack against a user, for example to reach deep tissues. It is preferable in such cases to apply the desired pressure when calculating the characteristic energy transfer rate and to keep it constant thereafter. However, it is not excluded that the conversion algorithms can include formulae that are dependent upon the pressure.

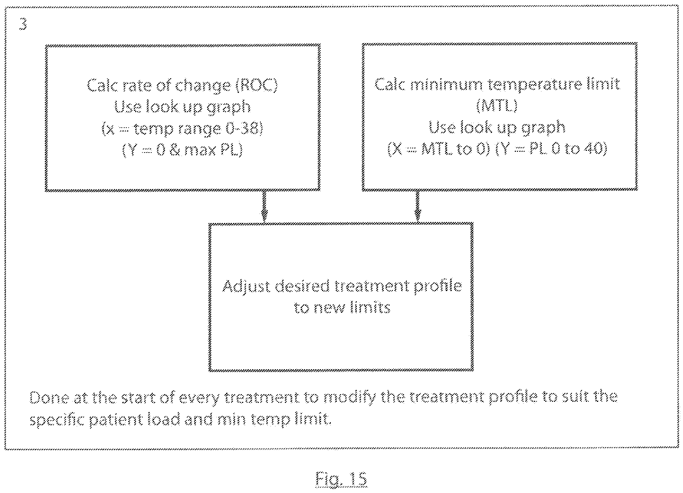

Accordingly, there is often a choice between varying the temperature of the thermoregulation fluid being supplied to the interface pack and/or varying the rate of flow of fluid. In some scenarios, for example as shown in Figure 15, the conversion algorithm includes a minimum and/or maximum temperature limit to minimise risk of harm to a user, meaning that variation solely of the temperature is not appropriate.

In some embodiments, there will be a plurality of possible conversion algorithms available, depending on which feed parameters are to be held fixed. In some embodiments, the user can select which conversion algorithm to use. In some embodiments, the control unit 56' is configured to select a conversion algorithm in accordance with predetermined criteria.

The control unit 56' is then operable to apply the conversion algorithm to a desired temperature profile in order to obtain one or more feed parameters, and to operate the system in accordance with the one or more feed parameters.

As the system is operating, the sensor 52' and the temperature sensor 54 constantly measure the temperature of thermoregulation fluid entering and leaving the interface pack (respectively the send temperature and the return temperature) and supply the data to the control unit 56'. The control unit 56' is operable to calculate therefrom the interface pack temperature, being the average of the send and return temperatures. If the control unit 56' determines that the interface pack temperature is not in accordance with the desired temperature profile, the control unit is operable to modify one or more feed parameters to bring the interface pack temperature into accordance with the desired temperature profile. If the interface pack temperature differs from the desired temperature by more than a threshold value, the control unit 56' can be operable to recalculate the characteristic energy transfer rate between the user and the pack and obtain a new conversion algorithm in the manner described above. This ensures that the system delivers safe volumes of energy, too much and tissue can be damaged, too little and a user can defeat the energy in the pack meaning the system would not be able to meet or hold the temperature as defined by the temperature profile.

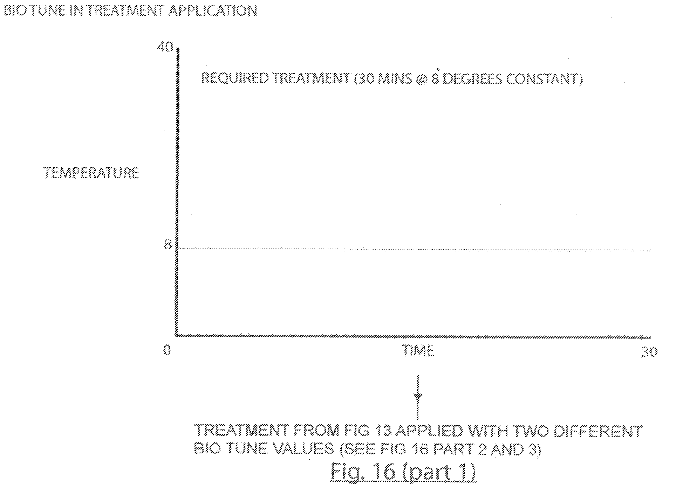

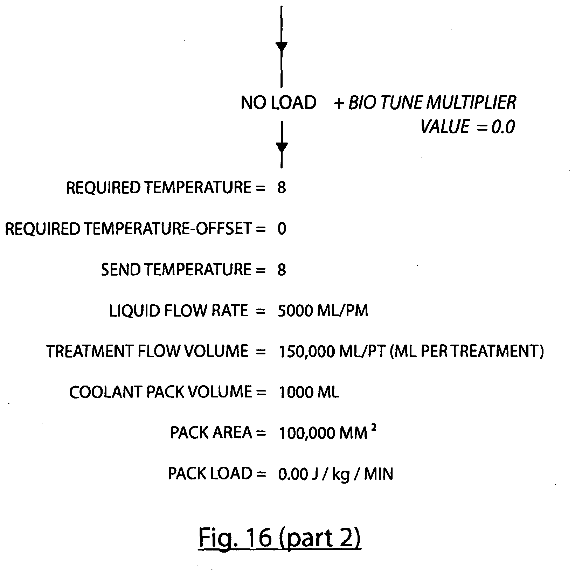

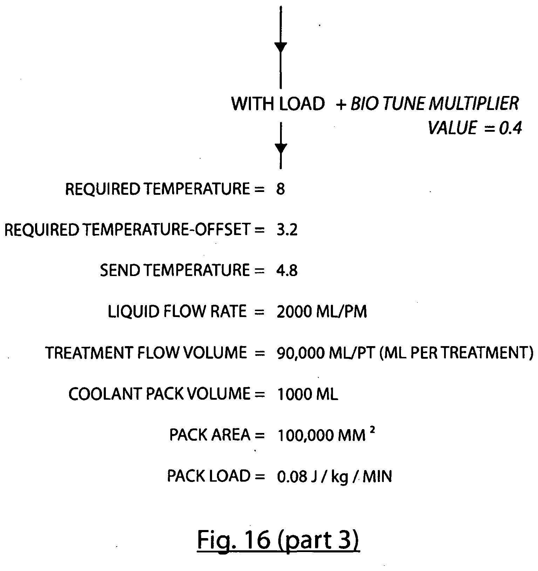

In the example shown in Figure 13, two tables of data are provided, one for the interface pack not placed on a user (no load), and one with the interface pack placed on a user (with load). The conversion algorithm in this example is with a liquid flow rate of 2000ml/min to apply a temperature multiplier of 0.4. As shown in Figure 16, an example of a desired temperature for the example of Figure 13 is to provide a constant 8°C for 30 minutes. The send temperature can be generated by mixing measured volumes over time so to achieve 8°C the system can send for 10 seconds at 1000ml/min at 4°C from the cold tank and 34°C for 2 seconds from the hot tank. However, using the conversion algorithm, a temperature offset is calculated by multiplying the desired temperature (8°C) by the temperature multiplier (0.4) to provide a temperature offset of 3.2°C. This is then subtracted from the desired temperature to provide a send temperature for the thermoregulation fluid of 4.8°C.

Although this example shows a desired temperature profile of a constant temperature, the system can equally use profiles including more complex curves of desired temperatures.

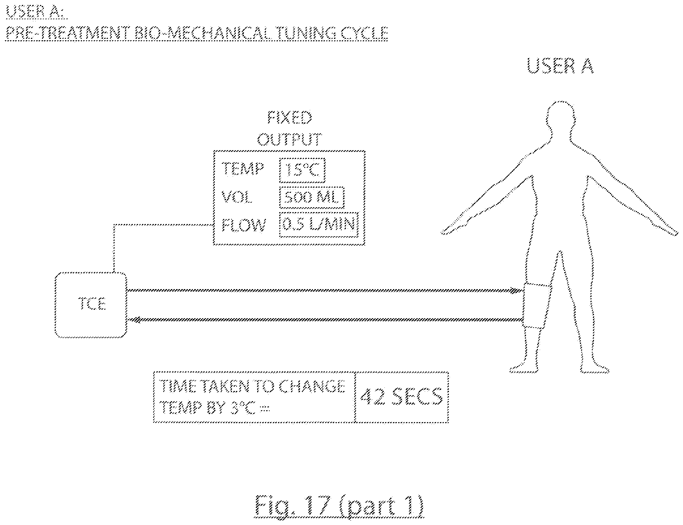

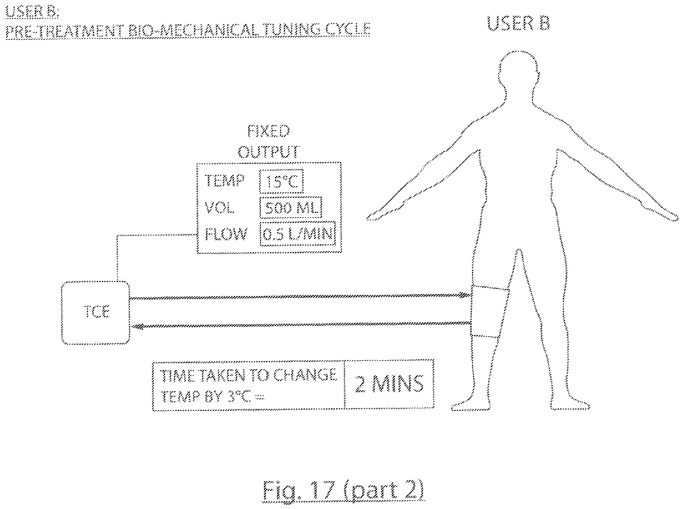

In the example shown in Figure 17, a characteristic energy transfer rate is calculated for both user A and user B using the same system. User A and user B each wear the interface pack which is supplied with thermoregulation fluid at a temperature of 15°C with a pack volume 500ml at a flow rate of 0.5L/min. The pack on user A changes by 3°C in 42 seconds whereas the pack on user B changes by 3°C in 2 minutes. Accordingly different conversion algorithms are calculated for users A and B.

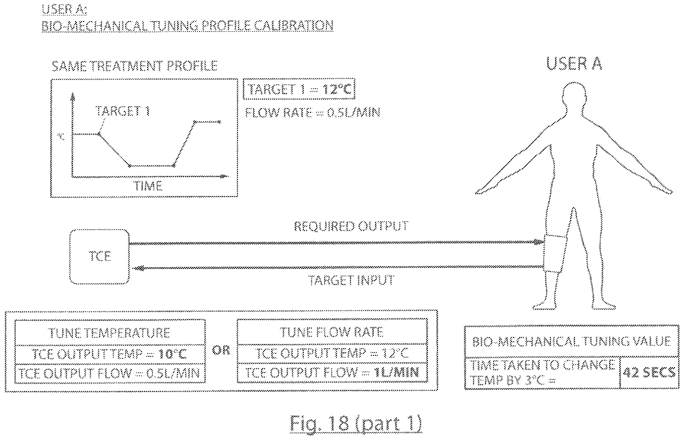

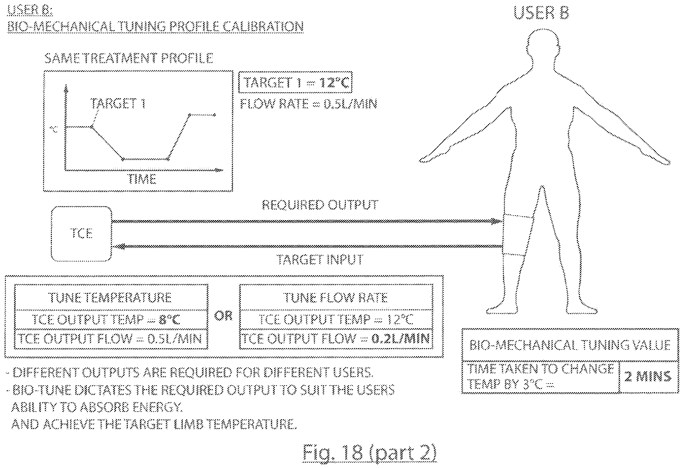

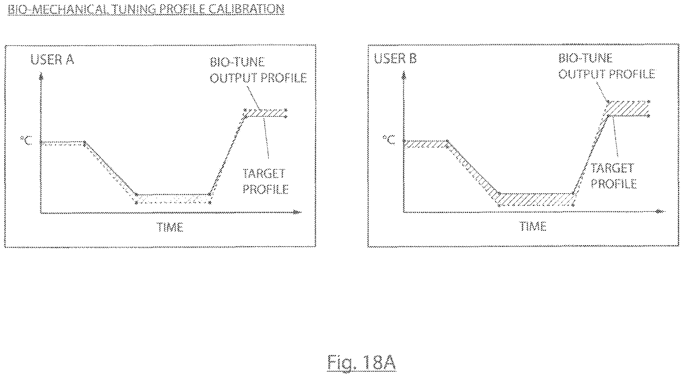

As can be seen from Figure 18, the same desired temperature profile, or treatment profile, is to be applied to each of users A and B, however different feed parameters are required.

As can be seen, in this example, there are two conversion algorithms available for each user. A first algorithm entitled 'tune temperature' provides a fixed flow rate of 0.5ml/min and provides a formula for the temperature of the thermoregulation fluid sent to the pack dependent upon the desired temperature profile. A second algorithm entitled 'tune flow rate' provides a fixed fluid temperature of 12°C and provides a formula for the flow rate of the thermoregulation fluid sent to the pack dependent upon the desired temperature profile.

In other words, in Figures 17 and 18, the same bio-mechanical tuning is performed on both user A and user B. A predefined temperature, volume and flow rate of fluid is output by the thermal compression engine (TCE) or manifold assembly, and the time required for the fluid temperature to change by a defined amount is recorded. User A has a shorter bio-mechanical tuning time, meaning he or she has less resistance to energy transfer. This could be due to smaller size, efficient metabolism or poorer fitness for example when compared with user B. User B has a longer bio-mechanical tuning time, meaning he or she has more resistance to energy transfer. This could be due to larger size, more efficient metabolism or greater fitness for example when compared with user A.

As user A offers less resistance, the bio-mechanical tuning profile is matched closer to the original target temperature profile. As user B offers more resistance, the bio-mechanical tuning profile deviates more from the original target temperature profile. Therefore, user B will require colder temperatures to be generated to achieve low targets and hotter temperatures to achieve high targets. This can be seen in Figure 18A.

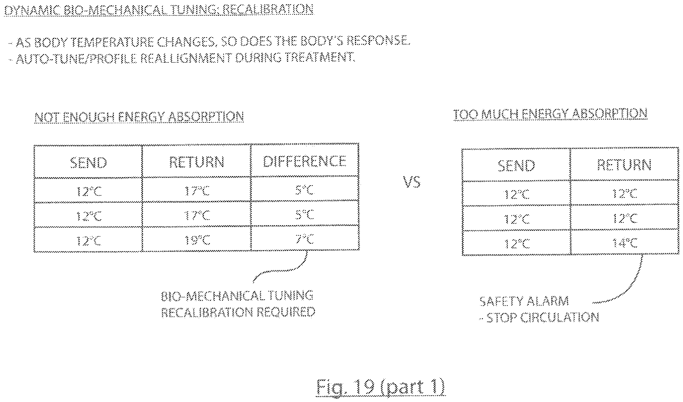

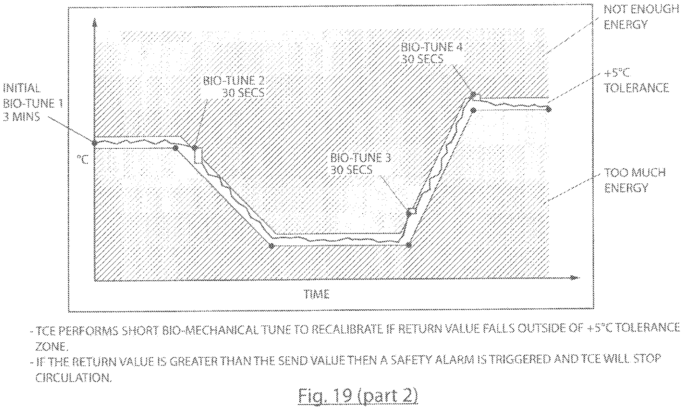

Figure 19 shows the continuation of the example of Figures 17 and 18 once treatment is in progress. In this example, the threshold for recalculating the characteristic energy transfer rate is 5°C. If there is a difference between the send temperature and the return temperature but that difference is less than or equal to 5°C, the control unit modifies the feed parameters to bring the interface pack temperature into accordance with the desired temperature profile.

In the table entitled 'not enough energy absorption' it can be seen that the send temperature is not succeeding in bringing the interface pack temperature to the desired temperature of 12°C. In other words, the pack is not absorbing enough energy from the user to reduce his or her temperature. Once the difference between the send and return temperatures exceeds 5°C, the system recalculates the characteristic energy transfer rate as described above.

In the table entitled 'too much energy absorption', in the first two rows the system is operating efficiently, keeping the temperature of the interface pack at the desired temperature of 12°C. However, in the third row, there is evidence of an error. The interface pack has for some reason absorbed too much energy, raising the temperature of the fluid by its return to 14°C. This causes a safety alarm to be triggered and the system will stop circulation of the fluid.

Alarms are activated at any time the profile does not met the real-time temperature average of the send and or return paths. This is done as it is assumed that if the desired profile contradicts the real time sensor output, the system will need to supply greater or lesser amounts of energy. In each case the alarms can be internal to the software and/or communicated to the user. Most alarms are ignored in purging and priming of the system as for example when starting new treatments there is a period of time of moving energy between the user and the system. However, in most scenarios the send temperature and return temperature will always be close; the send temperate will dictate any elevation or reduction in temperature and the return will follow. If there is a big differential either way and the unit is not running a contrast profile, then it is assumed that any big deflection between the two should trigger an alarm if that deflection is not present in the treatment profile.

The control unit can be programmed to perform the above described operations.

Figure 1A shows a patient 10A wearing various embodiments of a tubular cuff (which will be described in more detail with reference to later Figures). The various cuffs can be constructed so as to fit on the patient's arm 11A, elbow 12A, hand/wrist 13A, upper leg 14A, lower leg 15A, knee 16A, foot/ankle 17A or on both of the patient's legs 18A at the same time (the so-called "recovery trousers" embodiment). It will be appreciated that all of these examples are tubular in construction and therefore that the body part to be treated can be entirely surrounded by the tubular cuff by placing the body part inside the hollow part of the tube.

Turning to Figure 2A, tubular cuff 20A comprises a thermal chamber 22A which is defined by the cylindrical space between cylindrical inner wall 23A and cylindrical separator wall 24A. Pressure chamber 26A is defined by the space between separator wall 24A and outer wall 27A, such that pressure chamber 26A completely surrounds thermal chamber 22A and is concentric with it. The hollow space defined by tubular inner wall 23A is the space into which body part 21A is placed prior to treatment.

It can be seen that thermal chamber 22A is filled with open-celled foam 25A (specifically, 6mm reticulated polyurethane foam with a porosity of 0.39 pores per mm (10 pores per inch)) and the function of this will be described below.

Turning now to Figures 5A and 3A, these show a longitudinal cross-section of tubular cuff 20A (Figure 5A) and an enlarged part of Figure 5A (Figure 3A). It can be seen that inner wall 23A, separator wall 24A and outer wall 27A are formed from three tubes 31A, 32A and 33A respectively and that these are bonded together at either end of cuff 20A in order to terminate thermal chamber 22A and pressure chamber 26A. The tubes may be hollow to enable fluid to be conveyed along them or alternatively may simply be formed of a flexible solid material to function solely as a wall. In the example shown in Figures 3A and 5A, inner wall 23A and separator wall 24A are formed from for 80-150 micron polyurethane film and outer wall 27A is formed from 200 denier polyurethane-coated nylon.

As shown in Figures 3A, 5A, 6A and 7A, thermal chamber 22A and pressure chamber 26A are supplied with cooling/heating liquid and air respectively through the ends of tubular cuff 20A. Air feed tube 70A is connected to pressure chamber 26A via tubing port 75A and likewise liquid feed tube 71A, liquid return tube 72A and restricted liquid return tube 73A are connected to thermal chamber 22A via tubing ports 75A. Tubing ports 75A are welded in between the layflat layers. This configuration eliminates the need for tubing within the liquid chamber, which can distort the cuff.

There are two liquid returns: liquid return tube 72A at the bottom of cuff 20A and restricted liquid return tube 73A at the top of cuff 20A. Restricted liquid return tube 73A connects onto a smaller orifice size and functions as an exhaust to allow any air to escape from the thermal chamber 22A. This has been found to prevent air from restricting liquid flow and preventing liquid from exiting cuff 20A freely. Restricted liquid return tube 73A also allows cuff 20A to continue its operation even if held upside down or in the opposite direction to what is recommended without putting too much strain on the hardware which provides the cooling/heating liquid.

Cuff 20A may be fitted into outer jacket 30A having articulation area 100A and this is shown in more detail in Figure 8.

An alternative view of cuff 20A is shown in Figure 6A in which it can be seen that cuff 20A is formed from a series of concentric tubes fitted one inside the other, namely inner wall 23A, open celled foam 25A, separator wall 24A, outer wall 27A and outer jacket 30A from bottom to top (or from inside to outside) in Figure 6A. Figure 6A also shows air feed tube 70A, liquid feed tube 71A, liquid return tube 72A, restricted liquid return tube 73A and tubing ports 75A which are shown in more detail in Figure 7A.

In use, body part 21A is placed inside cuff 20A in the tubular space defined by inner wall 23A. Pressure chamber 26A is then partially inflated by pumping air into it through air feed tube 70A. Pressure chamber 26A is not fully inflated, but merely filled with sufficient air to enable cuff 20A to grip body part 21A. This also has the effect of compressing thermal chamber 22A against body part 21A in order to maximise the surface contact between inner wall 23A and the skin of patient 10A.

A key technical advantage of this construction is that any excess material formed of inner wall 23A, open-celled foam 25A and separator wall 24A is automatically accommodated by pressure chamber 26A to enable thermal chamber 22A to surround and contact body part 21A completely without flow of liquid within thermal chamber 22A being impeded. This is shown schematically in Figures 4a and 4b, in which Figure 4a shows cuff 20A under no compression and Figure 4b shows cuff 20A under compression caused by pressurised air in pressure chamber 26A compressing thermal chamber 22A against body part 21A. It can be seen that thermal chamber 22A completely contacts and surrounds body part 21A and that the parts of thermal chamber 22A which are in excess are accommodated within pressure chamber 26A in the form of ruckles 40A. It is preferable for the material defining thermal chamber 22A to be sufficiently thin to allow it to collapse into itself so that the excess material can move into the space of the pressure chamber 26A to form ruckle 40A.

Once thermal chamber 22A has been contoured around body part 21A, it is filled with cooling fluid as will now be described.

Thermal chamber 22A is filled with cooling fluid by injecting the fluid into liquid feed tube 71A. It then flows through open-celled foam 25A from one end of thermal chamber 22A to the other and then exits thermal chamber 22A via liquid return tube 72A.

The function of open-celled foam 25A is randomly and evenly to distribute the fluid throughout the thermal chamber 22A so that the cooling effect is applied evenly and completely over the surface area of body part 21A which is contacted by thermal chamber 22A. Foam 25A also takes up a large volume inside thermal chamber 22A, which means that not so much cooling fluid is required to 'submerse' the limb in liquid. It also maintains the shape and structure of the thermal chamber 22A, ensuring that fluid flow is not restricted to particular areas. Foam 25A can compress, deform and stretch to conform evenly around the body part. Different densities, thicknesses and weights of foam 25A can be used to improve coolant flow and thermal transfer.

Once thermal chamber 22A has been filled with cooling fluid, further pressure can be applied to the air in pressure chamber 26A in order to maximise compression of thermal chamber 22A against body part 21A. This can also be used to apply compression therapy to body part 21A, and this compression therapy can be varied by varying the pressure of air in pressure chamber 26A.

It will be appreciated that a heating fluid can be used instead of a cooling fluid for applying heat therapy to body part 21A.

When the therapy has been completed, flow of the cooling/heating fluid through thermal chamber 22A is ceased and air pressure is released by emptying the pressure chamber 26A so that cuff 20A can be removed from body part 21A.

As noted below, cuff 20A is fitted inside outer jacket 30A, preferably prior to being fitted onto body part 21A. Alternatively, outer jacket 30A can be applied over cuff 20A once it has been placed over body part 21A.

Outer jacket 30A is formed of any suitable textile such as ripstop nylon or coated fabric. The function of outer jacket 30A is to provide cylindrical support and to direct all compression forces inwardly onto body part 21A. It also restricts and controls the amount of outward ballooning from pressure chamber 26A.

As shown in Figure 8A, outer jacket 30 can include an elastic section 100A and a more rigid material 101A in order to provide an articulation area 105A around an articulated body part 21A. This allows for articulation of the body part and allows the device to bend laterally. It also allows the pressure chamber 26A to balloon and deform around the articulated joint whereas this ballooning and deforming is restricted by rigid material 101A around the straight/non-articulated parts of the limb.

Figure 9A shows an alternative example which provides for an articulation area 105A as in the embodiment of Figure 8A. In this example, the pressure chamber 26A is provided with plastic ligaments 110A which can stretch in a region 115A on the outside of the articulated body part to control deformation of the device as a whole. Ligaments 110A may alternatively be provided as a part of outer jacket 30A (not shown in Figure 9A).

It will be understood that different outer jackets 30A can be provided for different body parts 21A, depending upon the size, shape and articulation required and that these specific outer jackets 30A can be used with generic cuffs 20A.

Optionally, cuff 20A may include articulation coil 120A as shown in Figure 10A. This is formed of a helical coil of reinforcement wire and is housed in pressure chamber 26A. Its function is to provide radial support whilst allowing lateral flex 125A and thereby accommodate limb flexion.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A control system for a thermoregulation assembly, including:
a fluid manifold assembly (12') including a manifold input for receiving thermoregulation fluid from at least one source of thermoregulation fluid at a thermoregulation temperature, and a manifold output (26) for supplying thermoregulation fluid to an interface pack; the manifold assembly being operable to selectively provide thermoregulation fluid into a said interface pack via the manifold output in accordance with feed parameters;
a control unit (56') operable to obtain a characteristic energy transfer rate between a first user and a first such interface pack before a treatment plan is commenced, and to obtain a desired temperature profile and a conversion algorithm, the desired temperature profile providing a profile of desired interface pack temperature over time for the treatment plan and the conversion algorithm providing a formula for deriving one or more feed parameters for bringing the first interface pack to or maintaining the first interface pack at a desired temperature, the conversion algorithm being dependent upon the characteristic energy transfer rate;
wherein the control unit (56') is operable to calculate one or more feed parameters from the desired temperature profile and the conversion algorithm;
wherein the control unit (56') is operable to operate the fluid manifold assembly in accordance with the said one or more feed parameters; wherein the characteristic energy transfer rate is a difference between an absolute energy transfer rate of the first interface pack when applied to the first user, and an energy transfer rate of the first interface pack without a user.

2. A control system according to claim 1, wherein to obtain the characteristic energy transfer rate between the first user and the first interface pack the control unit is operable to operate the fluid manifold assembly with fixed predetermined feed parameters for a period of time and to calculate the characteristic energy transfer rate using measurements obtained during the said period of time.

3. A control system according to any preceding claim, wherein the fluid manifold assembly (12') includes a second input, the second input being for receiving thermoregulation fluid from a said interface pack, and an input temperature sensor (54) coupled to the control unit (56') and disposed to measure a return temperature of thermoregulation fluid received at the second input from a said interface pack;
wherein the control unit (56') is operable to repeatedly determine, based at least on the return temperature sensed by the input temperature sensor, an interface pack temperature;
wherein the control unit (56') is operable to modify one or more feed parameters in response to the interface pack temperature to bring the interface pack temperature into correspondence with the desired temperature profile;
wherein preferably:
the control system includes an output temperature sensor coupled to the control unit and disposed to measure a send temperature of thermoregulation fluid provided via the manifold output and the control unit (56') is operable to determine the interface pack temperature by calculating an average of the send temperature and the return temperature.

4. A control system according to any preceding claim, wherein the control unit (56') is operable to obtain a said conversion algorithm for each of a plurality of temperature bands, wherein the control unit (56') is operable to calculate the one or more feed parameters from the desired temperature profile and a conversion algorithm for the temperature band within which the system is operating.

5. A control system according to any preceding claim, wherein the manifold assembly (12') is operable to vary a temperature of fluid provided via the manifold output; wherein the manifold assembly (12') preferably includes the at least one source and the control unit (56') is operable to vary the temperature of the at least one source.

6. A control system according to claim 5 wherein the manifold input is operable selectively to receive thermoregulation fluid from a hot source of thermoregulation fluid and a cold source of thermoregulation fluid, the manifold assembly (12') including at least one mixer between the manifold input and the manifold output, the control unit being operable to control the at least one mixer to mix fluid received from the hot source and the cold source thereby to vary the temperature of fluid provided via the manifold output.

7. A control system according to any preceding claim wherein:
the manifold assembly (12') includes at least one controllable valve for selectively providing thermoregulation fluid via the manifold output; and/or
the control system includes a variable flow unit operable to control a flow rate and/or a pressure of fluid provided via the manifold output, the control unit (56') being operable to control the variable flow unit, the variable flow unit preferably including a pump; and/or
the one or more feed parameters include one or more of: a temperature of thermoregulation fluid provided via the manifold output, a flow rate of thermoregulation fluid provided via the manifold output, a pressure of thermoregulation fluid provided via the manifold output and a volume of thermoregulation fluid in a said interface pack.

8. A thermoregulation assembly including a control system according to any preceding claim; the thermoregulation assembly optionally including:
a pressure system including a source of pressure fluid and a pressure fluid outlet wherein the control unit is operable to control the pressure system to provide pressure fluid via the pressure fluid outlet with a predetermined pressure profile.

9. A program product for operating a thermoregulation assembly in accordance with a method, the assembly including:
a control unit (56');
an interface pack provided with at least one fluid path therein for the passage of thermoregulation fluid therethrough and a fluid inlet and a fluid outlet;
a fluid manifold assembly (12') including a manifold output attachable in fluid communication with the fluid inlet of the interface pack, the manifold assembly being operable to selectively provide thermoregulation fluid into the fluid inlet of the interface pack in accordance with feed parameters;
at least one source of thermoregulation fluid at a thermoregulation temperature coupled to a manifold input for supplying thermoregulation fluid to the manifold assembly;
the method including the steps of:
before commencing a treatment plan:
with the interface pack applied to a first user, operating the thermoregulation assembly with fixed predetermined feed parameters for a period of time; and
determining a characteristic energy transfer rate between the first user and the interface pack using measurements obtained during the said period of time;
obtaining a conversion algorithm dependent upon the characteristic energy transfer rate between the first user and the interface pack, the conversion algorithm providing a formula for deriving one or more feed parameters for bringing the interface pack to or maintaining the interface pack at a desired temperature;
obtaining a desired temperature profile, the desired temperature profile providing a profile of desired interface pack temperature;
calculating one or more feed parameters from the desired temperature profile and the conversion algorithm;
operating the fluid manifold assembly in accordance with the said one or more feed parameters; wherein the method further includes:
with the interface pack not applied to a user, operating the thermoregulation assembly with fixed predetermined feed parameters for a period of time;
determining a characteristic energy transfer rate of the pack without a user;
wherein determining the characteristic energy transfer rate between the first user and the interface pack includes subtracting the characteristic energy transfer rate of the pack without a user from an absolute energy transfer rate between the first user and the interface pack.

10. A program product according to claim 9, wherein the thermoregulation assembly includes an input temperature sensor (54) for sensing a return temperature of thermoregulation fluid leaving the fluid outlet of the interface pack and wherein the manifold assembly (12') is operable to provide thermoregulation fluid to the interface pack at a send temperature;
wherein determining the characteristic energy transfer rate between the first user and the interface pack includes:
operating the manifold assembly (12') to provide thermoregulation fluid to the interface pack with send temperature being fixed for a period of time;
operating the input temperature sensor (54) to measure the return temperature;
calculating a rate of change of temperature difference between the send temperature and the return temperature;
determining the characteristic energy transfer rate between the first user and the interface pack based on the said rate of change;
wherein determining the characteristic energy transfer rate between the first user and the interface pack based on the said rate of change preferably includes multiplying the said rate of change by the specific heat capacity of the thermoregulation fluid;
the method preferably including operating an output temperature sensor to measure the send temperature;
the method preferably including determining a said characteristic energy transfer rate between the first user and the interface pack for a plurality of temperature bands, including, for each temperature band, determining a said characteristic energy transfer rate between the first user and the interface pack wherein the send temperature is fixed at a characteristic temperature for the respective temperature band.

11. A program product according to claim 10, wherein determining the characteristic energy transfer rate between the first user and the interface pack includes:
operating the manifold assembly (12') to provide thermoregulation fluid to the interface pack with the send temperature being fixed for a first period of timewherein the interface pack is not applied to a user;
operating the input temperature sensor (54) to measure, preferably repeatedly, the return temperature during the first period;
calculating a first rate of change of temperature difference between the send temperature and the return temperature, the first rate of change corresponding to the first period;
determining the characteristic energy transfer rate of the interface pack without a user based on the first rate of change;
operating the manifold assembly (12') to provide thermoregulation fluid to the interface pack with the send temperature being fixed for a second period of time wherein the interface pack is applied to the first user;
operating the input temperature sensor (54) to measure, preferably repeatedly, the return temperature during the second period;
calculating a second rate of change of temperature difference between the send temperature and the return temperature, the second rate of change corresponding to the second period;
determining the characteristic energy transfer rate between the interface pack and the first user based on the second rate of change and including subtracting the characteristic energy transfer rate of the interface pack without a user.

12. A program product according to any of claims 9 to 11, wherein obtaining a conversion algorithm includes accessing a look-up table or graph and identifying a conversion algorithm corresponding to the characteristic energy transfer rate between the first user and the interface pack.

13. A program product according to any of claims 9 to 12, wherein the fluid manifold assembly includes a second input for receiving thermoregulation fluid from the interface pack and an input temperature sensor coupled to the control unit and disposed to measure a return temperature of thermoregulation fluid received at the second input from the interface pack; the method including:
repeatedly determining, based at least on the return temperature sensed by the input temperature sensor, an interface pack temperature;
modifying one or more feed parameters in response to the interface pack temperature to bring the interface pack temperature into correspondence with the desired temperature profile;
the program optionally including, if a difference between the interface pack temperature and the desired temperature profile exceeds a threshold value, re-determining the characteristic energy transfer rate between the first user and the interface pack and re-obtaining the conversion algorithm;
wherein, optionally:
the thermoregulation assembly includes an output temperature sensor coupled to the control unit and disposed to measure a send temperature of thermoregulation fluid provided to the interface pack and the method includes determining the interface pack temperature by calculating an average of the send temperature and the return temperature.

14. A control unit including a program product according to any of claims 9 to 13.

## Patentansprüche

1. Steuerungssystem für eine Thermoregulierungsanordnung, aufweisend:
eine Fluidverteileranordnung (12'), die einen Verteilereingang zum Aufnehmen von Thermoregulierungsfluid von mindestens einer Quelle von Thermoregulierungsfluid mit einer Thermoregulierungstemperatur, und einen Verteilerausgang (26) zum Liefern von Thermoregulierungsfluid an eine Schnittstellenpackung aufweist; wobei die Verteileranordnung dazu betreibbar ist, gemäß Einspeisungsparametern selektiv Thermoregulierungsfluid über den Verteilerausgang in die Schnittstellenpackung zu liefern;
eine Steuerungseinheit (56'), die dazu betreibbar ist, eine charakteristische Energietransferrate zwischen einem ersten Benutzer und einer ersten solchen Schnittstellenpackung zu erhalten, bevor ein Behandlungsplan begonnen wird, und ein gewünschtes Temperaturprofil und einen Umrechnungsalgorithmus zu erhalten, wobei das gewünschte Temperaturprofil ein Profil einer gewünschten Schnittstellenpackungstemperatur über die Zeit für den Behandlungsplan vorsieht und der Umrechnungsalgorithmus eine Formel zum Ableiten eines oder mehrerer Einspeisungsparameter zum Bringen der ersten Schnittstellenpackung auf eine gewünschte Temperatur oder Halten der ersten Schnittstellenpackung auf dieser vorsieht, wobei der Umrechnungsalgorithmus von der charakteristischen Energietransferrate abhängig ist;
wobei die Steuerungseinheit (56') dazu betreibbar ist, einen oder mehrere Einspeisungsparameter aus dem genannten gewünschten Temperaturprofil und dem Umrechnungsalgorithmus zu berechnen;
wobei die Steuerungseinheit (56') dazu betreibbar ist, die Fluidverteileranordnung gemäß dem einen oder den mehreren Einspeisungsparametern zu betrieben; wobei die charakteristische Energietransferrate eine Differenz zwischen einer absoluten Energietransferrate der ersten Schnittstellenpackung, wenn sie an dem ersten Benutzer angebracht ist, und einer Energietransferrate der ersten Schnittstellenpackung ohne einen Benutzer ist.

2. Steuerungssystem gemäß Anspruch 1, wobei zum Erhalten der charakteristischen Energietransferrate zwischen dem ersten Benutzer und der ersten Schnittstellenpackung die Steuerungseinheit dazu betreibbar ist, die Fluidverteileranordnung über einen Zeitraum mit festen vorbestimmten Einspeisungsparametern zu betreiben und die charakteristische Energietransferrate unter der Verwendung von Messungen zu berechnen, die während des Zeitraums erhalten wurden.

3. Steuerungssystem gemäß einem der vorhergehenden Ansprüche, wobei die Fluidverteileranordnung (12') einen zweiten Eingang, wobei der zweite Eingang zum Aufnehmen von Thermoregulierungsfluid von einer genannten Schnittstellenpackung ist, und einen Eingangstemperatursensor (54) aufweist, der mit der Steuerungseinheit (56') gekoppelt und dazu angeordnet ist, eine Rücklauftemperatur von Thermoregulierungsfluid zu messen, das an dem zweiten Eingang von einer genannten Schnittstellenpackung empfangen wird;
wobei die Steuerungseinheit (56') dazu betreibbar ist, wiederholt auf Basis mindestens der von dem Eingangstemperatursensor gemessenen Rücklauftemperatur eine Schnittstellenpackungstemperatur zu bestimmen;
wobei die Steuerungseinheit (56') dazu betreibbar ist, einen oder mehrere Einspeisungsparameter in Reaktion auf die Schnittstellenpackungstemperatur zu modifizieren, um die Schnittstellenpackungstemperatur in Übereinstimmung mit dem gewünschten Temperaturprofil zu bringen;
wobei vorzugsweise:
das Steuerungssystem einen Ausgangstemperatursensor aufweist, der mit der Steuerungseinheit gekoppelt und dazu angeordnet ist, eine Vorlauftemperatur über den Verteilerausgang gelieferten Thermoregulierungsfluids zu messen, und die Steuerungseinheit (56') dazu betreibbar ist, durch Berechnen eines Durchschnitts von Vorlauftemperatur und Rücklauftemperatur die Schnittstellenpackungstemperatur zu bestimmen.

4. Steuerungssystem gemäß einem der vorhergehenden Ansprüche, wobei die Steuerungseinheit (56') dazu betreibbar ist, einen genannten Umrechnungsalgorithmus für jedes von mehreren Temperaturbändern zu erhalten, wobei die Steuerungseinheit (56') dazu betreibbar ist, den einen oder die mehreren Einspeisungsparameter aus dem gewünschten Temperaturprofil und einem Umrechnungsalgorithmus für das Temperaturband zu berechnen, innerhalb dessen des System betrieben wird.

5. Steuerungssystem gemäß einem der vorhergehenden Ansprüche, wobei die Verteileranordnung (12') dazu betreibbar ist, eine Temperatur über den Verteilerausgang gelieferten Fluids zu variieren; wobei die Verteileranordnung (12') vorzugsweise die mindestens eine Quelle enthält und die Steuerungseinheit (56') dazu betreibbar ist, die Temperatur der mindestens einen Quelle zu variieren.

6. Steuerungssystem gemäß Anspruch 5, wobei der Verteilereingang dazu betreibbar ist, selektiv Thermoregulierungsfluid von einer warmen Quelle von Thermoregulierungsfluid und einer kalten Quelle von Thermoregulierungsfluid zu empfangen, wobei die Verteileranordnung (12') mindestens einen Mischer zwischen dem Verteilereingang und dem Verteilerausgang aufweist, wobei die Steuerungseinheit dazu betreibbar ist, den mindestens einen Mischer zum Mischen von Fluid steuern, das von der warmen Quelle und der kalten Quelle empfangen wird, um dadurch die Temperatur des über den Verteilerausgang gelieferten Fluids zu variieren.

7. Steuerungssystem gemäß einem der vorhergehenden Ansprüche, wobei:
die Verteileranordnung (12') mindestens ein steuerbares Ventil zum selektiven Liefern von Thermoregulierungsfluid über den Verteilerausgang aufweist; und/oder
das Steuerungssystem eine variable Strömungseinheit zum Steuern einer Strömungsrate und/oder eines Drucks über den Verteilerausgang gelieferten Fluids aufweist, wobei die Steuerungseinheit (56') dazu betreibbar ist, die variable Strömungseinheit zu betreiben, wobei die variable Strömungseinheit vorzugsweise eine Pumpe aufweist; und/oder
der eine oder die mehreren Einspeisungsparameter eines oder mehr aus dem Folgenden aufweisen: eine Temperatur über den Verteilerausgang gelieferten Thermoregulierungsfluids, eine Strömungsrate über den Verteilerausgang gelieferten Thermoregulierungsfluids, einen Druck über den Verteilerausgang gelieferten Thermoregulierungsfluids und ein Volumen über den Verteilerausgang gelieferten Thermoregulierungsfluids in einer genannten Schnittstellenpackung.

8. Steuerungssystem gemäß einem der vorhergehenden Ansprüche; wobei die Thermoregulierungsanordnung optional aufweist:
ein Drucksystem, das eine Quelle von Druckfluid und einen Druckfluidauslass aufweist, wobei die Steuerungseinheit dazu betreibbar ist, das Drucksystem zum Liefern von Druckfluid über den Druckfluidausgang mit einem vorbestimmten Druckprofil zu steuern.

9. Programmprodukt zum Betreiben einer Thermoregulierungsanordnung gemäß einem Verfahren, wobei die Anordnung aufweist:
eine Steuerungseinheit (56');
eine Schnittstellenpackung, die mit mindestens einem Fluidpfad darin für das Hindurchgelangen von Thermoregulierungsfluid und einem Fluideinlass und einem Fluidauslass ausgestattet ist;
eine Fluidverteileranordnung (12'), die einen Verteilerausgang aufweist, der in Fluidkommunikation mit dem Fluideinlass der Schnittstellenpackung anbringbar ist, wobei die Verteileranordnung dazu betreibbar ist, gemäß Einspeisungsparametern selektiv Thermoregulierungsfluid in den Fluideinlass der Schnittstellenpackung zu liefern;
mindestens eine Quelle für Thermoregulierungsfluid mit einer Thermoregulierungstemperatur, welche an einen Verteilereingang gekoppelt ist, um die Verteileranordnung mit Thermoregulierungsfluid zu versorgen,
wobei das Verfahren die folgenden Schritte aufweist:
vor Beginn eines Behandlungsplans:
während die Schnittstellenpackung an einem ersten Benutzer angebracht ist, Betreiben der Thermoregulierungsanordnung mit festen vorbestimmten Einspeisungsparametern während eines Zeitraums; und
Bestimmen einer charakteristischen Energietransferrate zwischen dem ersten Benutzer und der Schnittstellenpackung unter der Verwendung während des ersten Zeitraums erhaltener Messungen;
Erhalten eines Umrechnungsalgorithmus, der von der charakteristischen Energietransferrate zwischen dem ersten Benutzer und der Schnittstellenpackung abhängig ist, wobei der Umrechnungsalgorithmus eine Formel zum Ableiten eines oder mehrerer Einspeisungsparameter vorsieht, um die Schnittstellenpackung auf eine gewünschte Temperatur zu bringen oder sie auf dieser zu halten;
Erhalten eines gewünschten Temperaturprofils, wobei das gewünschte Temperaturprofil ein Profil einer gewünschten Schnittstellenpackungstemperatur vorsieht;
Berechnen eines oder mehrerer Einspeisungsparameter aus dem gewünschten Temperaturprofil und dem Umrechnungsalgorithmus;
Betreiben der Fluidverteileranordnung gemäß dem einen oder den mehreren Einspeisungsparametern; wobei das Verfahren ferner aufweist:
während die Schnittstellenpackung nicht an einem Benutzer angebracht ist, Betreiben der Thermoregulierungsanordnung mit festen vorbestimmten Einspeisungsparametern über einen Zeitraum;
Bestimmen einer charakteristischen Energietransferrate der Packung ohne einen Benutzer;
wobei das Bestimmen der charakteristischen Energietransferrate zwischen dem ersten Benutzer und der Schnittstellenpackung beinhaltet, dass die charakteristische Energietransferrate der Packung ohne einen Benutzer von einer absoluten Energietransferrate zwischen dem ersten Benutzer und der Schnittstellenpackung abgezogen wird.

10. Programmprodukt gemäß Anspruch 9,
wobei die Thermoregulierungsanordnung einen Eingangstemperatursensor (54) zum Erfassen einer Rücklauftemperatur des den Fluidauslass der Schnittstellenpackung verlassenden Thermoregulierungsfluids aufweist und wobei die Verteileranordnung (12') dazu betreibbar ist, Thermoregulierungsfluid mit einer Vorlauftemperatur an die Schnittstellenpackung zu liefern;
wobei das Bestimmen der charakteristischen Energietransferrate zwischen dem ersten Benutzer und der Schnittstellenpackung beinhaltet:
Betreiben der Verteileranordnung (12') zum Liefern von Thermoregulierungsfluid an die Schnittstellenpackung mit einer Vorlauftemperatur, die über einen Zeitraum fest bleibt;
Betreiben des Eingabetemperatursensors (54) zum Messen der Rücklauftemperatur;
Berechnen einer Änderungsrate einer Temperaturdifferenz zwischen der Vorlauftemperatur und der Rücklauftemperatur;
Bestimmen der charakteristischen Energietransferrate zwischen dem ersten Benutzer und der Schnittstellenpackung auf Basis der genannten Änderungsrate;
wobei das Bestimmen der charakteristischen Energietransferrate zwischen dem ersten Benutzer und der Schnittstellenpackung auf Basis der genannten Änderungsrate vorzugsweise ein Multiplizieren der genannten Änderungsrate mit der spezifischen Wärmekapazität des Thermoregulierungsfluids beinhaltet;
wobei das Verfahren vorzugsweise ein Betreiben eines Ausgangstemperatursensors zum Messen der Vorlauftemperatur beinhaltet;
wobei das Verfahren vorzugsweise ein Bestimmen einer genannten charakteristischen Energietransferrate zwischen dem ersten Benutzer und der Schnittstellenpackung für mehrere Temperaturbänder beinhaltet, einschließlich, für jedes Temperaturband, Bestimmen einer genannten charakteristischen Energietransferrate zwischen dem ersten Benutzer und der Schnittstellenpackung, wobei die Vorlauftemperatur für das jeweilige Temperaturband auf eine charakteristische Temperatur festgelegt wird.

11. Programmprodukt gemäß Anspruch 10,
wobei das Bestimmen der charakteristischen Energietransferrate zwischen dem ersten Benutzer und der Schnittstellenpackung aufweist:
Betreiben der Verteileranordnung (12') zum Liefern von Thermoregulierungsfluid an die Schnittstellenpackung, wobei die Vorlauftemperatur für einen ersten Zeitraum festgelegt ist, wobei die Schnittstellenpackung nicht an einem Benutzer angebracht ist;
Betreiben des Eingangstemperatursensors (54) zum vorzugsweise wiederholten Messen der Rücklauftemperatur während des ersten Zeitraums;
Berechnen einer ersten Änderungsrate der Temperaturdifferenz zwischen der Vorlauftemperatur und der Rücklauftemperatur, wobei die erste Änderungsrate dem ersten Zeitraum entspricht;
Bestimmen der charakteristischen Energietransferrate der Schnittstellenpackung ohne einen Benutzer auf Basis der ersten Änderungsrate;
Betreiben der Verteileranordnung (12') zum Liefern von Thermoregulierungsfluid an die Schnittstellenpackung, wobei die Vorlauftemperatur über einen zweiten Zeitraum festgelegt ist, während die Schnittstellenpackung an dem ersten Benutzer angebracht ist;
Betreiben des Eingangstemperatursensors (54) zum vorzugsweise wiederholten Messen der Rücklauftemperatur während des zweiten Zeitraums;
Berechnen einer zweiten Änderungsrate einer Temperaturdifferenz zwischen der Vorlauftemperatur und der Rücklauftemperatur, wobei die zweite Änderungsrate dem zweiten Zeitraum entspricht;
Bestimmen der charakteristischen Energietransferrate zwischen der Schnittstellenpackung und dem ersten Benutzer auf Basis der zweiten Änderungsrate und wobei enthalten ist, dass die charakteristische Energietransferrate der Schnittstellenpackung ohne einen Benutzer abgezogen wird.

12. Programmprodukt gemäß einem der Ansprüche 9 bis 11,
wobei ein Erhalten eines Umrechnungsalgorithmus ein Zugreifen auf eine Verweistabelle oder eine Kurve und ein Identifizieren eines Umrechnungsalgorithmus beinhaltet, der der charakteristischen Energietransferrate zwischen dem ersten Benutzer und der Schnittstellenpackung entspricht.

13. Programmprodukt gemäß einem der Ansprüche 9 bis 12,
wobei die Fluidverteileranordnung einen zweiten Eingang zum Aufnehmen von Thermoregulierungsfluid von der Schnittstellenpackung und einen Eingangstemperatursensor aufweist, der mit der Steuerungseinheit gekoppelt und dazu angeordnet ist, eine Rücklauftemperatur von an dem zweiten Eingang von der Schnittstellenpackung empfangenen Thermoregulierungsfluids zu messen; wobei das Verfahren aufweist:
wiederholtes Bestimmen einer Schnittstellenpackungstemperatur auf Basis mindestens der von dem Eingangstemperatursensor gemessenen Rücklauftemperatur;
Modifizieren eines oder mehrerer Einspeisungsparameter in Reaktion auf die Schnittstellenpackungstemperatur zum Bringen der Schnittstellenpackungstemperatur in Übereinstimmung mit dem gewünschten Temperaturprofil;
wobei das Programm optional beinhaltet, wenn eine Differenz zwischen der Schnittstellenpackungstemperatur und dem gewünschten Temperaturprofil einen Schwellenwert übersteigt, ein erneutes Bestimmen der charakteristischen Energietransferrate zwischen dem ersten Benutzer und der Schnittstellenpackung und ein erneutes Erhalten des Umrechnungsalgorithmus;
wobei optional:
die Thermoregulierungsanordnung einen Ausgangstemperatursensor aufweist, der mit der Steuerungseinheit gekoppelt und dazu angeordnet ist, die Vorlauftemperatur an die Schnittstellenpackung gelieferten Thermoregulierungsfluids zu messen, und das Verfahren ein Bestimmen der Schnittstellenpackungstemperatur durch Berechnen eines Durchschnitts aus Vorlauftemperatur und Rücklauftemperatur beinhaltet.

14. Steuerungseinheit, die ein Programmprodukt gemäß einem der Ansprüche 9 bis 13 aufweist.

## Revendications

1. Système de commande pour un ensemble de thermorégulation, comprenant :
un ensemble collecteur de fluide (12') comprenant une entrée de collecteur pour recevoir un fluide de thermorégulation en provenance d'au moins une source de fluide de thermorégulation à une température de thermorégulation, et une sortie de collecteur (26) pour alimenter en fluide de thermorégulation un bloc d'interface ; l'ensemble collecteur étant destiné à fournir de façon sélective un fluide de thermorégulation dans ledit bloc d'interface par le biais de la sortie de collecteur en fonction de paramètres d'alimentation ;
une unité de commande (56') destinée à obtenir un taux de transfert d'énergie caractéristique entre un premier utilisateur et un premier pareil bloc d'interface avant qu'un plan de traitement ne soit commencé, et à obtenir un profil de température souhaité et un algorithme de conversion, le profil de température souhaité fournissant un profil d'une température de bloc d'interface souhaitée au fil du temps pour le plan de traitement et l'algorithme de conversion fournissant une formule pour dériver un ou plusieurs paramètres d'alimentation pour amener le premier bloc d'interface à une température souhaitée ou le maintenir à cette dernière, l'algorithme de conversion étant fonction du taux de transfert d'énergie caractéristique ;
dans lequel l'unité de commande (56') est destinée à calculer un ou plusieurs paramètres d'alimentation à partir du profil de température souhaité et de l'algorithme de conversion ;
dans lequel l'unité de commande (56') est destinée à faire fonctionner l'ensemble collecteur de fluide en fonction dudit ou des dits paramètres d'alimentation ; dans lequel le taux de transfert d'énergie caractéristique est une différence entre un taux de transfert d'énergie absolu du premier bloc d'interface lorsqu'il est appliqué à un premier utilisateur, et un taux de transfert d'énergie du premier bloc d'interface sans utilisateur.

2. Système de commande selon la revendication 1, dans lequel pour obtenir le taux de transfert d'énergie caractéristique entre le premier utilisateur et le premier bloc d'interface, l'unité de commande est destinée à faire fonctionner l'ensemble collecteur de fluide avec des paramètres d'alimentation fixes prédéterminés pendant une certaine période de temps et à calculer le taux de transfert d'énergie caractéristique à l'aide de mesures obtenues pendant ladite période de temps.

3. Système de commande selon l'une quelconque des revendications précédentes, dans lequel l'ensemble collecteur de fluide (12') comprend une seconde entrée, la seconde entrée étant destinée à recevoir un fluide de thermorégulation en provenance d'un dit bloc d'interface, et un capteur de température d'entrée (54) couplé à l'unité de commande (56') et disposé de sorte à mesurer une température de retour d'un fluide de thermorégulation reçu au niveau de la seconde entrée en provenance d'un dit bloc d'interface ;
dans lequel l'unité de commande (56') est destinée à déterminer de façon répétée, en se basant au moins sur la température de retour détectée par le capteur de température d'entrée, une température de bloc d'interface ;
dans lequel l'unité de commande (56') est destinée à modifier un ou plusieurs paramètres d'alimentation en réponse à la température de bloc d'interface pour amener la température de bloc d'interface en correspondance avec le profil de température souhaité ;
dans lequel de préférence :
le système de commande comprend un capteur de température de sortie couplé à l'unité de commande et disposé de sorte à mesurer une température d'envoi d'un fluide de thermorégulation fourni par le biais de la sortie de collecteur et l'unité de commande (56') est destinée à déterminer la température de bloc d'interface en calculant une moyenne de la température d'envoi et de la température de retour.

4. Système de commande selon l'une quelconque des revendications précédentes, dans lequel l'unité de commande (56') est destinée à obtenir un dit algorithme de conversion pour chaque bande de température d'une pluralité de bandes de température, dans lequel l'unité de commande (56') est destinée à calculer le ou les paramètres d'alimentation à partir du profil de température souhaité et d'un algorithme de conversion pour la bande de température dans laquelle le système fonctionne.

5. Système de commande selon l'une quelconque des revendications précédentes, dans lequel l'ensemble collecteur (12') est destiné à faire varier une température d'un fluide fourni par le biais de la sortie de collecteur ; dans lequel l'ensemble collecteur (12') comprend de préférence la ou les sources et l'unité de commande (56') est destinée à faire varier la température de la ou des sources.

6. Système de commande selon la revendication 5, dans lequel l'entrée de collecteur est destinée de façon sélective à recevoir un fluide de thermorégulation en provenance d'une source chaude de fluide de thermorégulation et d'une source froide de fluide de thermorégulation, l'ensemble collecteur (12') comprenant au moins un mélangeur entre l'entrée de collecteur et la sortie de collecteur, l'unité de commande étant destinée à commander le ou les mélangeurs pour mélanger un fluide reçu de la source chaude et de la source froide pour faire, de ce fait, varier la température d'un fluide fourni par le biais de la sortie de collecteur.

7. Système de commande selon l'une quelconque des revendications précédentes, dans lequel :
l'ensemble collecteur (12') comprend au moins une vanne réglable pour fournir de façon sélective un fluide de thermorégulation par le biais de la sortie de collecteur ; et/ou
le système de commande comprend une unité à écoulement variable destinée à réguler un débit et/ou une pression d'un fluide fourni par le biais de la sortie de collecteur, l'unité de commande (56') étant destinée à commander l'unité à écoulement variable, l'unité à écoulement variable comprenant de préférence une pompe ; et/ou
le ou les paramètres d'alimentation comprennent : une température d'un fluide de thermorégulation fourni par le biais de la sortie de collecteur et/ou un débit d'un fluide de thermorégulation fourni par le biais de la sortie de collecteur et/ou une pression d'un fluide de thermorégulation fourni par le biais de la sortie de collecteur et/ou un volume d'un fluide de thermorégulation dans un dit bloc d'interface.

8. Ensemble de thermorégulation comprenant un système de commande selon l'une quelconque des revendications précédentes ; l'ensemble de thermorégulation comprenant facultativement :
un système de pression comprenant une source de fluide de pression et un orifice de sortie de fluide de pression, dans lequel l'unité de commande est destinée à commander le système de pression de sorte à fournir un fluide de pression par le biais de l'orifice de sortie de fluide de pression avec un profil de pression prédéterminé.

9. Produit de programme pour faire fonctionner un ensemble de thermorégulation en fonction d'un procédé, l'ensemble comprenant :
une unité de commande (56') ;
un bloc d'interface comportant au moins un trajet de fluide en son sein pour le passage d'un fluide de thermorégulation à travers ce dernier ainsi qu'un orifice d'entrée de fluide et un orifice de sortie de fluide ;
un ensemble collecteur de fluide (12') comprenant un orifice de sortie de collecteur pouvant être fixé en communication fluidique avec l'orifice d'entrée de fluide du bloc d'interface, l'ensemble collecteur étant destiné à fournir de façon sélective un fluide de thermorégulation dans l'orifice d'entrée de fluide du bloc d'interface en fonction de paramètres d'alimentation ;
au moins une source de fluide de thermorégulation à une température de thermorégulation couplée à un orifice d'entrée de collecteur pour fournir un fluide de thermorégulation à l'ensemble collecteur ;
le procédé comprenant les étapes consistant :
avant de commencer un plan de traitement :
avec le bloc d'interface appliqué à un premier utilisateur, à faire fonctionner l'ensemble de thermorégulation avec des paramètres d'alimentation fixes prédéterminés pendant une certaine période de temps ;
à déterminer un taux de transfert d'énergie caractéristique entre le premier utilisateur et le bloc d'interface en utilisant les mesures obtenues pendant ladite période de temps ;
à obtenir un algorithme de conversion en fonction du taux de transfert d'énergie caractéristique entre le premier utilisateur et le bloc d'interface, l'algorithme de conversion fournissant une formule pour dériver un ou plusieurs paramètres d'alimentation pour amener le bloc d'interface à une température souhaitée ou le maintenir à cette dernière ;
à obtenir un profil de température souhaité, le profil de température souhaité fournissant un profil de température de bloc d'interface souhaitée ;
à calculer un ou plusieurs paramètres d'alimentation à partir du profil de température souhaité et de l'algorithme de conversion ;
à faire fonctionner l'ensemble collecteur de fluide en fonction dudit ou desdits paramètres d'alimentation ; dans lequel le procédé consiste en outre :
avec le bloc d'interface qui n'est pas appliqué à un utilisateur, à faire fonctionner l'ensemble de thermorégulation avec des paramètres d'alimentation fixes prédéterminés pendant une certaine période de temps ;
à déterminer un taux de transfert d'énergie caractéristique du bloc sans utilisateur ;
dans lequel la détermination du taux de transfert d'énergie caractéristique entre le premier utilisateur et le bloc d'interface consiste à soustraire le taux de transfert d'énergie caractéristique du bloc sans utilisateur d'un taux de transfert d'énergie absolu entre le premier utilisateur et le bloc d'interface.

10. Produit de programme selon la revendication 9,
dans lequel l'ensemble de thermorégulation comprend un capteur de température d'entrée (54) pour détecter une température de retour d'un fluide de thermorégulation quittant l'orifice de sortie de fluide du bloc d'interface et dans lequel l'ensemble collecteur (12') est destiné à fournir un fluide de thermorégulation au bloc d'interface à une température d'envoi ;
dans lequel la détermination du taux de transfert d'énergie caractéristique entre le premier utilisateur et le bloc d'interface consiste :
à faire fonctionner l'ensemble collecteur (12') pour fournir un fluide de thermorégulation au bloc d'interface, la température d'envoi étant fixée pendant une certaine période de temps ;
à faire fonctionner le capteur de température d'entrée (54) pour mesurer la température de retour ;
à calculer un taux de variation d'une différence de température entre la température d'envoi et la température de retour ;
à déterminer le taux de transfert d'énergie caractéristique entre le premier utilisateur et le bloc d'interface en se basant sur ledit taux de variation ;
dans lequel la détermination du taux de transfert d'énergie caractéristique entre le premier utilisateur et le bloc d'interface en se basant sur ledit taux de variation consiste de préférence à multiplier ledit taux de variation par la capacité thermique spécifique du fluide de thermorégulation ;
le procédé consistant de préférence à faire fonctionner un capteur de température de sortie pour mesurer la température d'envoi ;
le procédé consistant de préférence à déterminer un dit taux de transfert d'énergie caractéristique entre le premier utilisateur et le bloc d'interface pour une pluralité de bandes de température, y compris, pour chaque bande de température, à déterminer un dit taux de transfert d'énergie caractéristique entre le premier utilisateur et le bloc d'interface dans lequel la température d'envoi est fixée à une température caractéristique pour la bande de température respective.

11. Produit de programme selon la revendication 10,
dans lequel la détermination du taux de transfert d'énergie caractéristique entre le premier utilisateur et le bloc d'interface consiste :
à faire fonctionner l'ensemble collecteur (12') pour fournir un fluide de thermorégulation au bloc d'interface, la température d'envoi étant fixée pendant une première période de temps pendant laquelle le bloc d'interface n'est pas appliqué à un utilisateur ;
à faire fonctionner le capteur de température d'entrée (54) pour mesurer, de préférence de manière répétée, la température de retour pendant la première période ;
à calculer un premier taux de variation d'une différence de température entre la température d'envoi et la température de retour, le premier taux de variation correspondant à la première période ;
à déterminer le taux de transfert d'énergie caractéristique du bloc d'interface sans utilisateur en se basant sur le premier taux de variation ;
à faire fonctionner l'ensemble collecteur (12') pour fournir un fluide de thermorégulation au bloc d'interface, la température d'envoi étant fixée pendant une seconde période de temps pendant laquelle le bloc d'interface est appliqué au premier utilisateur ;
à faire fonctionner le capteur de température d'entrée (54) pour mesurer, de préférence de manière répétée, la température de retour pendant la seconde période ;
à calculer un second taux de variation d'une différence de température entre la température d'envoi et la température de retour, le second taux de variation correspondant à la seconde période ;
à déterminer le taux de transfert d'énergie caractéristique entre le bloc d'interface et le premier utilisateur en se basant sur le second taux de variation et consistant à soustraire le taux de transfert d'énergie caractéristique du bloc d'interface sans utilisateur.

12. Produit de programme selon l'une quelconque des revendications 9 à 11,
dans lequel l'obtention d'un algorithme de conversion consiste à avoir accès à une table de conversion ou à un graphe et à identifier un algorithme de conversion correspondant au taux de transfert d'énergie caractéristique entre le premier utilisateur et le bloc d'interface.

13. Produit de programme selon l'une quelconque des revendications 9 à 12,
dans lequel l'ensemble collecteur de fluide comprend une seconde entrée pour recevoir un fluide de thermorégulation en provenance du bloc d'interface, et un capteur de température d'entrée couplé à l'unité de commande et disposé de sorte à mesurer une température de retour d'un fluide de thermorégulation reçu au niveau de la seconde entrée en provenance du bloc d'interface ; le procédé consistant :
à déterminer de manière répétée, en se basant au moins sur la température de retour détectée par le capteur de température d'entrée, une température de bloc d'interface ;
à modifier un ou plusieurs paramètres d'alimentation en réponse à la température de bloc d'interface pour amener la température de bloc d'interface en correspondance avec le profil de température souhaité ;
le programme consistant facultativement si une différence entre la température de bloc d'interface et le profil de température souhaité dépasse une valeur de seuil, à déterminer à nouveau le taux de transfert d'énergie caractéristique entre le premier utilisateur et le bloc d'interface et à obtenir à nouveau l'algorithme de conversion ;
dans lequel, facultativement :
l'ensemble de thermorégulation comprend un capteur de température de sortie couplé à l'unité de commande et disposé de sorte à mesurer une température d'envoi d'un fluide de thermorégulation fourni au bloc d'interface et le procédé consiste à déterminer la température de bloc d'interface en calculant une moyenne de la température d'envoi et de la température de retour.

14. Unité de commande comprenant un produit de programme selon l'une quelconque des revendications 9 à 13.
